# EUROPEAN PATENT SPECIFICATION

(11) **EP 3 828 542 B1**
(45) Date of publication and mention of the grant of the patent: **06.12.2023**
(21) Application number: 20204675.1
(22) Date of filing: 29.10.2020
(51) Int. Cl.: G01N 33/50, C08G 65/34, C08G 65/333

(54) **LAYERED BODY**
SCHICHTKÖRPER
CORPS STRATIFIÉ

(30) Priority: 15.11.2019 JP 2019207225
(43) Date of publication of application: 02.06.2021
(73) Proprietor: Ricoh Company, Ltd., Tokyo 143-8555 (JP)
(72) Inventor: IWASHITA, Natsuko, Tokyo, 143-8555 (JP); YAGINUMA, Hidekazu, Tokyo, 143-8555 (JP); SHIONOIRI, Momoko, Tokyo, 143-8555 (JP); SAMESHIMA, Tatsuya, Tokyo, 143-8555 (JP); KUBO, Chihiro, Tokyo, 143-8555 (JP); SATOH, Naoki, Tokyo, 143-8555 (JP); YAMAZAKI, Takehiro, Tokyo, 143-8555 (JP)
(74) Representative: J A Kemp LLP

(56) References cited:
- EP-A1- 3 006 559
- EP-A1- 3 269 755
- EP-B1- 3 006 559
- WO-A1-2014/126228
- WO-A1-2019/071135
- US-A1- 2012 122 949
- MATSUNAGA TAKURO ET AL: "Structure Characterization of Tetra-PEG Gel by Small-Angle Neutron Scattering", MACROMOLECULES, vol. 42, no. 4, 24 February 2009 (2009-02-24), pages 1344-1351, XP055795319, Washington DC United States ISSN: 0024-9297, DOI: 10.1021/ma802280n Retrieved from the Internet: URL:https://pubs.acs.org/doi/pdf/10.1021/m a802280n>
- GAO BOTAO ET AL: "Quantitating distance-dependent, indirect cell-cell interactions with a multilayered phospholipid polymer hydr", BIOMATERIALS, ELSEVIER, AMSTERDAM, NL, vol. 35, no. 7, 12 December 2013 (2013-12-12), pages 2181-2187, XP028807697, ISSN: 0142-9612, DOI: 10.1016/J.BIOMATERIALS.2013.11.060

## Description

### BACKGROUND OF THE INVENTION

### Field of the Invention

The present invention relates to a layered body used in cell culture techniques in vitro.

### Description of the Related Art

Examples of *in vitro* cell culture techniques include a technique called co-culture, in which a plurality of types of cells are cultured in the same container. One common method of co-culture is a contact method in which a plurality of types of cells are cultured simultaneously on the same plane, and another is a non-contact method in which a plurality of types of cells are cultured, for example, separated up and down, through a membrane called an insert, through which cells are not allowed to pass through. The contact method can evaluate effects of direct contact between cells of different types and paracrine effects of proteins secreted by cells into a medium on other cells, while the non-contact method can evaluate paracrine effects by proteins secreted by cells in an insert.

In the contact method, each cell cannot be recognized when detecting the paracrine effect, and it is necessary to use a separate marker to distinguish cells in order to examine the paracrine effect on each cell. On the other hand, in the non-contact method, an effect on each cell is distinguishable because cells are separated by an insert, but a detected paracrine effect is diluted and weakened because proteins (signal factors) secreted by the cells diffuse into a medium, which is problematic.

As a conventional technique for arranging a plurality of types of cells in a non-contact state, (Non-Patent Document 1) discloses a configuration in which one type of multi-layered cell layer is arranged in an insert and a spheroid in which cells of another type are made spherical with hydrogel on the back side of the insert for the purpose of forming a mucus layer by co-culture. Patent Document 1 discloses a configuration in which cells are made into sheets and gelatin hydrogel particles are sandwiched between the cell sheets to form a three-layered cell sheet for the purpose of maintaining cell survival.

However, it is difficult to detect the paracrine effect on each cell with the above-described conventional techniques with high intensity.

### Related Art Documents

### [Patent Document]

[Patent Document 1] WO2014/192909

### Non-Patent Document

[Non-Patent Document 1] Quaozhi Lu, et al., "An In Vitro Model for the Ocular Surface and Tear Film System," SCIENTIFIC REPORTS, 7:6163.

Accordingly, an object of the present invention is to provide a layered body of cells that is used in a co-culture technique and that is capable of recognizing a paracrine effect of each cell and detecting the effect with high intensity.

### SUMMARY OF THE INVENTION

In order to solve the above-described problem, the layered body of the present invention is a layered body having a layered structure in which a gel layer containing a hydrogel is disposed between at least two cell layers containing cells of different types from each other, wherein the hydrogel is a multi-branched polymer hydrogel formed by a reaction of: Liquid A containing a multi-branched polymer A, the polymer containing, as a backbone, a polyethylene glycol containing at least three branches, the branches containing one or more electrophilic functional groups in at least one of a side chain(s) and an end(s); and Liquid B containing a multi-branched polymer B, the polymer containing, as a backbone, a polyethylene glycol containing at least three branches, the branches containing one or more nucleophilic functional groups in at least one of a side chain(s) and an end(s), the concentration of components derived from the multi-branched polymers A and B in the hydrogel is from 0.6 % by weight to 8% by weight, and the thickness is from 0.02 mm to 2 mm.

The layered body of the present invention can be used for co-culture of a plurality of types of cells, in which a paracrine effect for each cell can be recognized. A layered body by which such a paracrine effect is exhibited with high intensity can be provided.

### BRIEF DESCRIPTION OF THE DRAWINGS

FIG. 1 is a schematic diagram illustrating a layered body according to a first embodiment.
FIG. 2 is a schematic diagram illustrating a procedure for producing the layered body according to the first embodiment.
FIG. 3 is a schematic diagram illustrating a layered body according to a second embodiment.
FIG. 4 is a schematic diagram illustrating a layered body according to a third embodiment.
FIG. 5 is a schematic diagram illustrating a layered body according to a fourth embodiment.
FIG. 6 is a schematic diagram illustrating a procedure for producing the layered body according to the fourth embodiment.
FIG. 7 is a schematic diagram illustrating a layered body according to a fifth embodiment.
FIG. 8 is a schematic diagram illustrating a layered body according to a sixth embodiment.
FIG. 9 is a schematic diagram illustrating a layered body according to a seventh embodiment.
FIG. 10 is a schematic diagram illustrating a layered body according to an eighth embodiment.
FIG. 11 is a schematic diagram illustrating a layered body according to a ninth embodiment.
FIG. 12 is a sectional view of a layered body in Example 1.
FIG. 13 is a graph illustrating the expression levels of a type I collagen α1 chain in Example 2 and Comparative Example 1.
FIG. 14 is a graph illustrating measurement results of the cell viability in a layered body of Comparative Example 2.
FIG. 15 is a graph illustrating measurement results of the cell viability in a layered body of Example 3.
FIG. 16 is a graph illustrating measurement results of the fluorescence intensity of dextran with a molecular weight of 500 kDa in Example 1.
FIG. 17 is a graph illustrating measurement results of the luminescence intensity in Example 4.
FIG. 18 is a graph illustrating measurement results of the luminescence intensity in Reference Example 4.
FIG. 19 is a confocal microscope image of a hydrogel support substrate produced in Example 7.
FIG. 20 is a confocal microscope image of a layered structure of the layered body produced in Example 7.
FIG. 21 is a graph illustrating measurement results of the cell viability in Examples 8 and 9.
FIG. 22 is a confocal microscopic image of a section of the layered body of Example 10.
FIG. 23 is a graph illustrating measurement results of the luminescence intensity of Examples 13 and 14.
FIG. 24 is a graph illustrating measurement results of the diffusion amount for different molecular weights in Reference Example 5.
FIG. 25 is a graph illustrating measurement results of the luminescence intensity of Examples 15 and 16.

### DESCRIPTION OF THE EMBODIMENTS

The present invention is described below in detail according to embodiments.

Since the embodiments described below are preferred embodiments of the present invention, various technically preferred limitations are given to the embodiments. However, as long as there is no description indicating limitation of the present invention in the following description, the scope of the present invention is not limited to these modes.

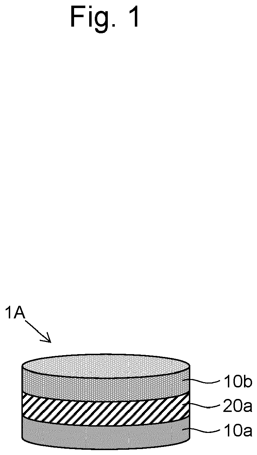

A first embodiment of the present invention will be described based on FIG. 1. A layered body 1A of FIG. 1 is schematically configured with a layered structure in which a gel layer 20a containing a hydrogel is disposed between two cell layers 10a and 10b containing different types of cells from each other. Each element constituting the layered body 1A will be described in detail below.

The type and the like of the cells contained in the cell layers 10a and 10b are not limited, and may be appropriately selected depending on the purpose. In terms of taxonomy, the cells may be, for example, eukaryotic cells, prokaryotic cells, multicellular organism cells, unicellular organism cells, or the like. Any cells may be used. The cells are preferably adhesive cells having cell adhesiveness which is enough to allow adhesion of the cells to each other and to prevent isolation of the cells from each other as long as the cells are not subjected to a physicochemical treatment.

The adherent cells are not limited, and may be appropriately selected depending on the purpose. Examples of the adhesive cells include differentiated cells and undifferentiated cells.

Examples of the differentiated cells include hepatocytes as parenchymal cells of the liver; stellate cells; Kupffer cells; vascular endothelial cells; endothelial cells such as sinusoidal endothelial cells and corneal endothelial cells; fibroblasts; osteoblasts; osteoclasts; periodontal membrane-derived cells; epidermal cells such as epidermal keratinocytes; tracheal epithelial cells; gastrointestinal epithelial cells; cervical epithelial cells; epithelial cells such as corneal epithelial cells; mammary cells; pericytes; muscle cells such as smooth muscle cells and cardiomyocytes; kidney cells; pancreatic Langerhans islet cells; nerve cells such as peripheral nerve cells and optic nerve cells; chondrocytes; and bone cells. The adhesive cells may be primary cells directly collected from a tissue or an organ, or may be subcultured cells obtained after several passages.

The undifferentiated cells are not limited, and may be appropriately selected depending on the purpose. Examples of the undifferentiated cells include pluripotent stem cells, such as embryonic stem cells, which are undifferentiated cells, and mesenchymal stem cells, which have pluripotency; unipotent stem cells such as vascular endothelial progenitor cells, which have unipotency; and iPS cells.

The density of cells in the cell layers 10a and 10b is not limited, and the cells may be directly bound to each other. The cells are preferably seeded at a seeding density of, for example, 5,000 to 60,000 cells/cm².

The cell layers 10a and 10b may contain materials such as various extracellular substrates or media as needed, which may be filled between the cells. The extracellular substrate is not limited, and may be appropriately selected depending on the purpose. For example, extracellular substrate proteins such as collagen, laminin, fibronectin, elastin, fibrin, proteoglycans, hyaluronic acid, heparan sulfate proteoglycans, or chondroitin sulfate proteoglycans, glycoproteins, and proliferation/growth factors such as hepatocyte growth factor, fibroblast growth factor, or nerve growth factor depending on the cell may be used. Any one type of these cells may be used individually, or two or more types of these cells may be used in combination. These are known to promote cell adhesion, proliferation, and differentiation, and can trigger changes in cell morphology when these cells are contained in the cell layer.

The culture medium is a solution containing components required for formation and maintenance of the three-dimensional structure. The medium prevents drying, and controls the external environment including the osmotic pressure. The culture medium is not limited, and may be appropriately selected from known culture media depending on the intended use. For a three-dimensional structure which does not need to be constantly immersed in a culture medium, such as skin whose surface is exposed to air, the culture medium may be removed as appropriate.

The culture medium is not limited, and may be appropriately selected depending on the purpose. Examples of the culture medium include various culture media classified according to the composition, such as natural media, semi-synthetic media, and synthetic media; and various culture media classified according to the shape, such as semi-solid media, liquid media, and powder media. Any one of these culture media may be used individually, or two or more types of these culture media may be used in combination. In cases where the cells are derived from an animal, any culture medium for use in animal cell culture may be used.

The culture medium for use in animal cell culture is not limited, and may be appropriately selected depending on the purpose. Examples of the culture medium include Dulbecco's Modified Eagle's Medium (D-MEM), Ham's F12 medium (Ham's Nutrient Mixture F12), D-MEM/F12 medium, McCoy's 5A medium, Eagle's MEM medium (Eagle's Minimum Essential Medium (EMEM)), αMEM medium (alpha Modified Eagle's Minimum Essential Medium; αMEM), MEM medium (Minimum Essential Medium), RPMI 1640 medium, Iscove's Modified Dulbecco's Medium (IMDM), MCDB131 medium, William's medium E, IPL 41 medium, Fischer's medium, StemPro 34 (manufactured by Invitrogen), X-VIVO 10 (manufactured by Cambrex Corporation), X-VIVO 15 (manufactured by Cambrex Corporation), HPGM (manufactured by Cambrex Corporation), StemSpan H3000 (manufactured by StemCell Technologies Inc.), StemSpan SFEM (manufactured by StemCell Technologies Inc.), Stemline II (manufactured by Sigma-Aldrich), QBSF-60 (manufactured by Quality Biological, Inc.), StemPro hESC SFM (manufactured by Invitrogen), Essential 8 (registered trademark) medium (manufactured by Gibco), mTeSR-1 or -2 medium (manufactured by StemCell Technologies Inc.), Repro FF or Repro FF2 (manufactured by ReproCELL Inc.), PSGro hESC/iPSC medium (manufactured by System Biosciences, Inc.), NutriStem (registered trademark) medium (manufactured by Biological Industries), CSTI-7 medium (manufactured by Cell Science & Technology Institute, Inc.), MesenPRO RS medium (manufactured by Gibco), MF-Medium (registered trademark) mesenchymal stem cell growth medium (manufactured by Toyobo Co., Ltd.), Sf-900II (manufactured by Invitrogen), and Opti-Pro (manufactured by Invitrogen). Any one type of these culture media may be used individually, or two or more of these culture media may be used in combination.

The carbon dioxide concentration in the culture medium is not limited, and may be appropriately selected depending on the purpose. The carbon dioxide concentration is preferably from 2% to 5%, more preferably from 3% to 4%. In cases where the carbon dioxide concentration is from 2% to 5%, the cells can be appropriately cultured.

The hydrogel contained in the gel layer 20a is a multi-branched polymer hydrogel formed by a reaction of: Liquid A containing a multi-branched polymer A, the polymer containing, as a backbone, a polyethylene glycol containing at least three branches, the branches containing one or more electrophilic functional groups in at least one of a side chain(s) and an end(s); and Liquid B containing a multi-branched polymer B, the polymer containing, as a backbone, a polyethylene glycol containing at least three branches, the branches containing one or more nucleophilic functional groups in at least one of a side chain(s) and an end(s). Each component is described below.

### <Multi-branched Polymer Containing Polyethylene Glycol as Backbone>

The multi-branched polymer containing a polyethylene glycol as a backbone is a polymer containing three or more polyethylene glycol branches, wherein molecules of the polymer cross-link to each other to form a network structure. In particular, four-branched polymers form homogeneous network structures, and gels having a four-branched polyethylene glycol backbone are generally known as Tetra-PEG gels. A Tetra-PEG has a network structure formed by cross-end coupling reaction between two kinds of four-branched polymers each containing an electrophilic functional group or a nucleophilic functional group in at least one of a side chain(s) and an end(s).

A past study has reported that a Tetra-PEG gel has an ideal homogeneous network structure (Matsunaga T, et al., Macromolecules, Vol.42, No.4, pp.1344-1351 (2009)). A Tetra-PEG gel can be simply prepared on site by mixing of two polymer liquids, and the gelation time can be controlled by adjusting the pH and the polymer concentration of each polymer liquid (which corresponds to each of Liquid A and Liquid B in the present invention). By allowing the Liquid A and the Liquid B to react, and then allowing gelation of the liquids to form a Tetra-PEG gel, a gel layer 20a can be produced. Since the gel layer 20a contains a polyethylene glycol as a major component, the gel layer has excellent biocompatibility, and a paracrine effect in which proteins secreted from a cell layer 10a act on another cell layer 10b (or vice versa) can be evaluated at a high concentration.

The total number of the electrophilic functional group(s) in the polymer in Liquid A and the nucleophilic functional group(s) in the polymer in Liquid B is preferably not less than 6. Although these functional groups may be present in one or both of a side chain(s) and an end(s) of each polymer, the functional groups are preferably present in an end(s) of each polymer. The content of the electrophilic functional group in the polymer in Liquid A may be higher than the content of the nucleophilic functional group in the polymer in Liquid B in the composition. Alternatively, the content of the nucleophilic functional group in the polymer in Liquid B may be higher than the content of the electrophilic functional group in the polymer in Liquid A in the composition. In a preferred mode, two or more kinds of combination of Liquid A and Liquid B having different compositions may be used to once form two or more kinds of gel precursors having different compositions, and the gel precursors may be further cross-linked to obtain a gel having a three-dimensional structure.

The electrophilic functional group contained in the multi-branched polymer in Liquid A is preferably maleimidyl, which is an active ester group. When necessary, in addition to the maleimidyl, the polymer may contain N-hydroxy-succinimidyl (NHS), sulfosuccinimidyl, phthalimidyl, imidazoyl, acryloyl, nitrophenyl, or the like. Those skilled in the art can select and employ other known active ester groups as appropriate. Among the multi-branched polymer molecules contained in Liquid A, the composition of the electrophilic functional group may be either the same or different. The composition is preferably the same. In cases where the functional group composition is the same, reactivity with the nucleophilic functional group forming the cross-link is homogeneous, and therefore a gel having a homogeneous spatial structure can be easily obtained.

The nucleophilic functional group contained in the multi-branched polymer in Liquid B is preferably thiol. When necessary, in addition to the thiol, the polymer may contain amino, -CO₂PhNO₂ (wherein Ph represents o-, m-, or p-phenylene), or the like. Those skilled in the art can select and employ various nucleophilic functional groups as appropriate. Among the multi-branched polymer molecules contained in Liquid B, the composition of the nucleophilic functional group may be either the same or different. The composition is preferably the same. In cases where the functional group composition is the same, reactivity with the electrophilic functional group forming the cross-link is homogeneous, and therefore a gel having a homogeneous spatial structure can be easily obtained.

Preferred specific examples of a multi-branched polymer containing one or more maleimidyl groups in at least one of a side chain(s) and an end(s) and containing a polyethylene glycol as a backbone include, but are not limited to, compounds represented by the following Formula (I), which contains four polyethylene glycol backbone branches, and maleimidyl groups at the ends.

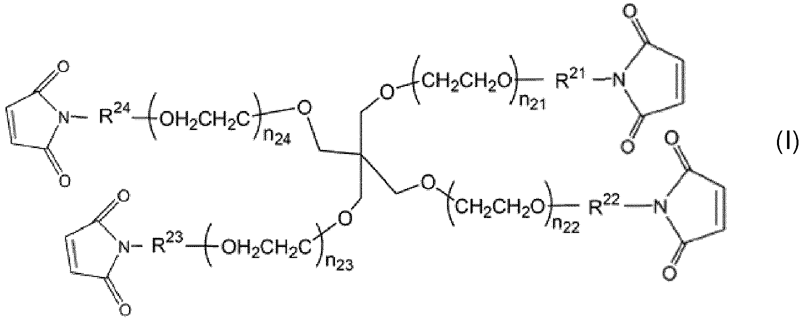

In the Formula (I), each of n₂₁ to n₂₄ may be either the same or different. As the values of n₂₁ to n₂₄ become close to each other, the gel can have a more homogeneous spatial structure, which is preferred because of a higher strength of the gel. n₂₁ to n₂₄ especially preferably have the same value. In cases where the values of n₂₁ to n₂₄ are too high, the gel has a lower strength, while in cases where the values of n₂₁ to n₂₄ are too low, the gel can be hardly formed due to steric hindrance of the compound. Thus, each of n₂₁ to n₂₄ appropriately has a value of from 5 to 300, preferably from 20 to 250, more preferably from 30 to 180, still more preferably from 45 to 115, especially preferably from 45 to 55. The multi-branched polymer in Liquid A has a weight average molecular weight of preferably from 5 × 10³ to 5 × 10⁴, more preferably from 7.5 × 10³ to 3 × 10⁴, still more preferably from 1 × 10⁴ to 2 × 10⁴.

In the Formula (I), R²¹ to R²⁴ are linker portions that link the functional groups to the core portion. Although R²¹ to R²⁴ may be either the same or different, R²¹ to R²⁴ are preferably the same from the viewpoint of producing a gel having a homogeneous spatial structure and a high strength. In Formula (I), R²¹ to R²⁴ are the same or different, and examples of R²¹ to R²⁴ include C₁-C₇ alkylene, C₂-C₇alkenylene, -NH-R²⁵-, -CO-R²⁵-, -R²⁶-OR²⁷-, -R²⁶-NH-R²⁷-, -R²⁶-CO₂-R²⁷-, -R²⁶-CO₂-NH-R²⁷-, -R²⁶-CO-R²⁷-, -R²⁶-NH-CO-R²⁷-, and - R²⁶-CO-NH-R²⁷-. Here, R²⁵ represents C₁-C₇ alkylene; R²⁶ represents C₁-C₃ alkylene; and R²⁷ represents C₁-C₅ alkylene.

Preferred specific examples of a multi-branched polymer containing one or more thiol groups in at least one of a side chain(s) and an end(s) and containing a polyethylene glycol as a backbone include, but are not limited to, compounds represented by the following Formula (II), which contains four polyethylene glycol backbone branches, and thiol groups at the ends.

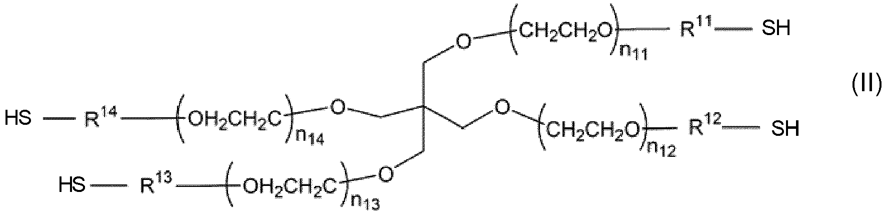

In the Formula (II), each of n₁₁ to n₁₄ may be either the same or different. As the values of n₁₁ to n₁₄ become close to each other, the gel can have a more homogeneous spatial structure, which is preferred because of a higher strength of the gel. n₁₁ to n₁₄ especially preferably have the same value. In cases where the values of n₁₁ to n₁₄ are too high, the gel has a lower strength, while in cases where the values of n₁₁ to n₁₄ are too low, the gel can be hardly formed due to steric hindrance of the compound. Thus, each of n₁₁ to n₁₄ has a value of preferably from 25 to 250, more preferably from 35 to 180, still more preferably from 50 to 115, especially preferably from 50 to 60. The multi-branched polymer in Liquid B has a weight average molecular weight of preferably from 5 × 10³ to 5 × 10⁴, more preferably from 7.5 × 10³ to 3 × 10⁴, still more preferably from 1 × 10⁴ to 2 × 10⁴.

In the Formula (II), R¹¹ to R¹⁴ are linker portions that link the functional groups to the core portion. Although R¹¹ to R¹⁴ may be either the same or different, R¹¹ to R¹⁴ are preferably the same from the viewpoint of producing a gel having a homogeneous spatial structure and a high strength. In Formula (II), R¹¹ to R¹⁴ are the same or different, and examples of R¹¹ to R¹⁴ include C₁-C₇ alkylene, C₂-C₇alkenylene, -NH-R¹⁵-, -CO-R¹⁵-, -R¹⁶-O-R¹⁷-, -R¹⁶-NH-R¹⁷- -R¹⁶-CO₂-R¹⁷-, -R¹⁶-CO₂-NH-R¹⁷-, -R¹⁶-CO-R¹⁷-, -R¹⁶-NH-CO-R¹⁷-, and - R¹⁶-CO-NH-R¹⁷-. Here, R¹⁵ represents C₁-C₇ alkylene; R¹⁶ represents C₁-C₃ alkylene; and R¹⁷ represents C₁-C₅ alkylene.

Here, "C₁-C₇ alkylene" means an alkylene group which may be branched and which has from 1 to 7 carbon atoms, and means a linear C₁-C₇ alkylene group, or a C₂-C₇ alkylene group having one or more branches (having from 2 to 7 carbon atoms including the carbon atoms in the branch(es)). Examples of the C₁-C₇ alkylene include methylene, ethylene, propylene, and butylene. More specific examples of the C₁-C₇ alkylene include -CH₂-, - (CH₂)₂-, -(CH₂)₃-, -CH(CH₃)-, -(CH₂)₃-, -(CH(CH₃))₂-, -(CH₂)₂-CH(CH₃)-, -(CH₂)₃-CH(CH₃)-, -(CH₂)₂-CH(C₂H₅)-, -(CH₂)₆-, -(CH₂)₂-C(C₂H₅)₂-, and -(CH₂)₃C(CH₃)₂CH₂-.

"C₂-C₇ alkenylene" means a linear or branched alkenylene group having from 2 to 7 carbon atoms, and containing one or more double bonds in the chain. Examples of the C₂-C₇ alkenylene include divalent groups containing a double bond, which groups are formed by elimination of from 2 to 5 hydrogen atoms from adjacent carbon atoms of the alkylene group.

In the present description, the alkylene group and the alkenylene group may contain one or more arbitrary substituents. Examples of such substituents include, but are not limited to, alkoxy, halogen atoms (any of a fluorine atom, chlorine atom, bromine atom, and iodine atom), amino, mono- or di-substituted amino, substituted silyl, acyl, and aryl. In cases where two or more substituents are contained, those substituents may be either the same or different.

As defined in the present description, in cases where a functional group "may have a substituent(s)", the type(s), the substitution site(s), and the number of the substituent(s) are not limited. In cases where two or more substituents are contained, the substituents may be either the same or different. Examples of the substituents include, but are not limited to, alkyl, alkoxy, hydroxy, carboxyl, halogen atoms, sulfo, amino, alkoxycarbonyl, and oxo. These substituents may further contain a substituent.

### <Type and Concentration of Buffer>

Each of Liquid A and Liquid B forming a gel layer 20a preferably contains an appropriate buffer in addition to the multi-branched polymer component containing a polyethylene glycol as a backbone. Examples of the buffer include phosphate buffer, citrate buffer, citrate-phosphate buffer, acetate buffer, borate buffer, tartrate buffer, Tris buffer, Tris-HCl buffer, phosphate buffered saline, citrate-phosphate buffered saline, and cell culture media. The buffer in Liquid A and the buffer in Liquid B may be either the same or different. Each of the buffer in Liquid A and the buffer in Liquid B may be a mixture of two or more kinds of buffers.

In cases where the concentration of the buffer is too low, the pH buffering capacity in the solution is low, and therefore a gel having a high strength cannot be produced. On the other hand, in cases where the buffer concentration is too high, mixing of the multi-branched polymer component contained in Liquid A and containing a polyethylene glycol as a backbone, with the multi-branched polymer component contained in Liquid B and containing a polyethylene glycol as a backbone, is inhibited. Therefore, a gel having a high strength cannot be produced. Thus, the concentration of the buffer in each of Liquid A and Liquid B is preferably within the range of from 20 mM to 200 mM from the viewpoint of production of a gel having a homogeneous structure and a high strength.

### <pH of Buffer, and Concentration of Multi-branched Polymer Containing Polyethylene Glycol as Backbone>

As described above, the gelation time can be controlled by adjusting the pH of the buffer and the concentration of the multi-branched polymer which is contained in each of Liquid A and Liquid B and which contains a polyethylene glycol as a backbone. More specifically, a buffer is used such that the pH of each of Liquid A and Liquid B is preferably adjusted to 5 to 10. The concentration of the multi-branched polymer which is contained in each of Liquid A and Liquid B and which contains a polyethylene glycol as a backbone is preferably adjusted within the range of from 0.3% by mass to 20% by mass. The pH of each of Liquid A and Liquid B is preferably from 6 to 10, and the concentration of the multi-branched polymer which is contained in each of Liquid A and Liquid B and which contains a polyethylene glycol as a backbone is preferably from 1.7% by mass to 20% by mass. The concentration of components derived from multi-branched polymers A and B in a hydrogel is preferably from 0.6% by weight to 8% by weight.

In an acidic solution having a pH of less than 5 in which the concentration of the multi-branched polymer containing a polyethylene glycol as a backbone is less than 0.3% by mass, the nucleophilic functional group is likely to be in a cationic state, leading to repulsion from each other. As a result, reactivity between the nucleophilic functional group in the cationic state and the electrophilic functional group in the other polymer component decreases. On the other hand, in cases where the concentration of the multi-branched polymer containing a polyethylene glycol as a backbone is higher than 20% by mass, the viscosity of a liquid is high, which is not desirable. In an alkaline solution having a pH of more than 10, reactivity between the nucleophilic functional group and the electrophilic functional group is too high, so that the gelation time is abnormally short. Therefore, each polymer cannot be sufficiently dispersed throughout the gel, and the gel becomes fragile as a result. Thus, the solution is not suitable.

### <Viscosity of Liquid A and Liquid B>

Since formation of the gel layer 20a is difficult when the viscosity of Liquid A and Liquid B is too high, specifically, the viscosity of Liquid A and Liquid B at 25°C is preferably 30 mPa·s or less.

### <Molar Ratio between Nucleophilic Functional Group and Electrophilic Functional Group>

Liquid A and Liquid B are preferably mixed together such that the molar ratio between the nucleophilic functional group and the electrophilic functional group is within the range of from 0.5:1 to 1.5:1. Since the functional groups react with each other at 1:1 to form a cross-link, the closer the mixing molar ratio to 1:1, the more preferred. For obtaining a hydrogel having a high strength, the ratio is especially preferably within the range of from 0.8:1 to 1.2:1.

### <Other Components>

Liquid A and Liquid B may contain other components, if necessary. The other components are not limited, and may be appropriately selected depending on the purpose. Examples of the other components include culture media, cross-linking agents, pH adjusters, antiseptics, and antioxidants.

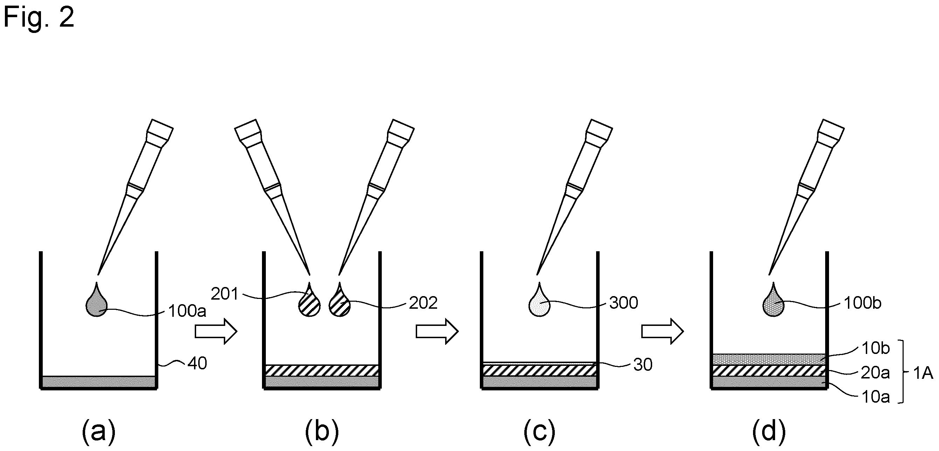

The procedure for producing a layered body 1A according to the present embodiment will be described based on FIG. 2. As illustrated in FIG. 2, a layered body 1A is usually formed in a culture container 40. Such a culture container is made of a substrate material that may serve as a base or a support needed for the formation and maintenance of the layered body 1A, and examples of the container include resin, glass, and metal. The shape of the substrate can be not only flat but also perforated, meshed, uneven, honeycomb, or the like, and can be selected appropriately depending on the application. A multi-well type culture plate, culture membrane, or the like, which is highly light-permeable and does not exhibit autofluorescence/luminescence, is preferably used. FIG. 2 is an example in which each layer of the cell layer 10a or the like covers the entire bottom surface of the culture container 40, but the layered body 1A is not necessarily always attached to the inner surface of the culture container 40. In the example in FIG. 2, the top of the culture container 40 is open for the loading and unloading of media or drugs, but the culture container may be a closed system and capable of reflux culture or a tip-shaped culture container with a microfluidic channel.

When preparing the layered body 1A, first, as illustrated in FIG. 2A, a cell suspension 100a for forming the cell layer 10a is added to the culture container 40. The cell suspension 100a contains cells suspended in a culture medium. The added cell suspension 100a is incubated as appropriate, and the culture medium is removed to form the cell layer 10a. Subsequently, Liquid A 201 containing a multi-branched polymer A and Liquid B containing a multi-branched polymer B are added sequentially or simultaneously to form the gel layer 20a.

The layered body 1A is prepared by forming the cell layer 10b on top of the gel layer 20a. As illustrated in FIG. 2C, in order to secure the adhesion of the cell layer 10b to the gel layer 20a, it is preferable to add an extracellular substrate solution 300 on top of the gel layer 20a and allow an extracellular substrate 30 to adhere to the gel layer 20. After adding the extracellular substrate solution 300, it is preferable to perform incubation as appropriate and remove the redundant extracellular substrate 30. The same type of extracellular substrate as the extracellular substrate that can be included in the cell layers 10a and 10b may be used, or a different type of extracellular substrate may be used.

Then, a cell suspension 100b for forming the cell layer 10b is added. After the addition, incubation is performed as appropriate, and the culture medium is removed to form the cell layer 10b to obtain the layered body 1A.

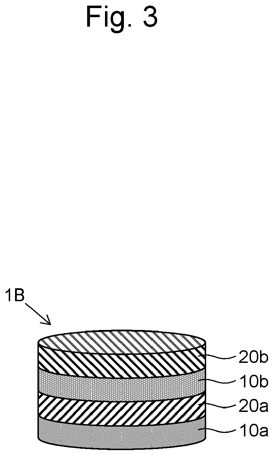

A second embodiment of the present invention will be described based on FIG. 3. The layered body 1B of FIG. 3 has a layered structure in which a gel layer 20a including a hydrogel is disposed between two cell layers 10a and 10b containing different types of cells from each other, as in the first embodiment, and furthermore, a gel layer 20b including a hydrogel is disposed on top of the cell layer 10b. The hydrogel included in the gel layer 20b is preferably the same hydrogel as the multi-branched polymer hydrogel included in the gel layer 20a, but the hydrogel may be of a different type.

In this second embodiment of the layered body 1B, the upper gel layer 20b has a barrier property and can protect the cell layer 10b. As described below, by containing a drug or a bio-derived liquid factor in the gel layer 20b, the above-described drug and the like can be released slowly from the gel layer 20b to the cell layer 10b. The layered body 1B can be prepared, for example, by preparing the layered body 1A in accordance with the first embodiment, and adding Liquid A containing the multi-branched polymer A and Liquid B containing the multi-branched polymer B to the top of the layered body 1A to form the gel layer 20b.

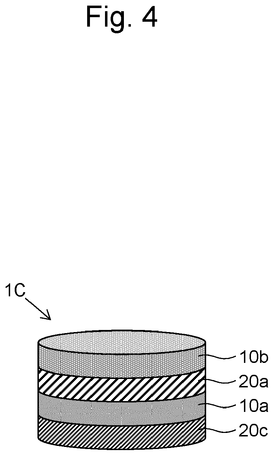

A third embodiment of the present invention will now be described based on FIG. 4. In the layered body 1C of FIG. 4, a gel layer 20c containing a hydrogel is further disposed at the bottom (lower portion of the cell layer 10a) of the three-layered layered body according to the first embodiment, which includes a gel layer 20a containing a hydrogel, between two cell layers 10a and 10b containing different types of cells from each other. The hydrogel contained in the gel layer 20c is preferably the same hydrogel as the multi-branched polymer hydrogel contained in the gel layer 20a, but the hydrogel may be of a different type.

The layered body 1C according to the third embodiment has an advantage that the gel layer 20c is in contact with the bottom of the culture container, and therefore the layered body 1C can be easily separated and collected from the culture container by peeling the gel layer 20c from the bottom of the culture container. The layered body 1C can be prepared, for example, by preparing the culture container 40 as in the first embodiment, sequentially or simultaneously adding Liquid A 201 containing the multi-branched polymer A and Liquid B containing the multi-branched polymer B, and allowing the gel layer 20c to gel, and then forming the cell layer 10a, the gel layer 20a, and the cell layer 10b on the gel layer 20c in the same procedure as in the first embodiment.

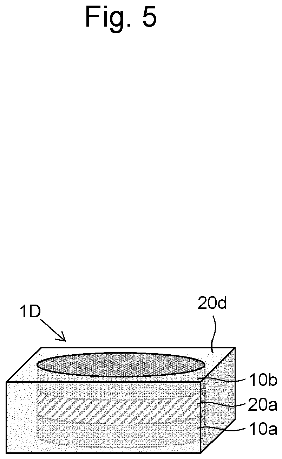

Next, a fourth embodiment of the present invention will be described based on FIG. 5. In a layered body 1D of FIG. 5, the side face of the three-layered layered body according to the first embodiment, which has a gel layer 20a containing a hydrogel disposed between two cell layers 10a and 10b containing different types of cells from each other, is further covered by a gel 20d containing a hydrogel. The hydrogel contained in the gel layer 20d is preferably the same hydrogel as the multi-branched polymer hydrogel contained in the gel layer 20a, but the hydrogel may be of a different type.

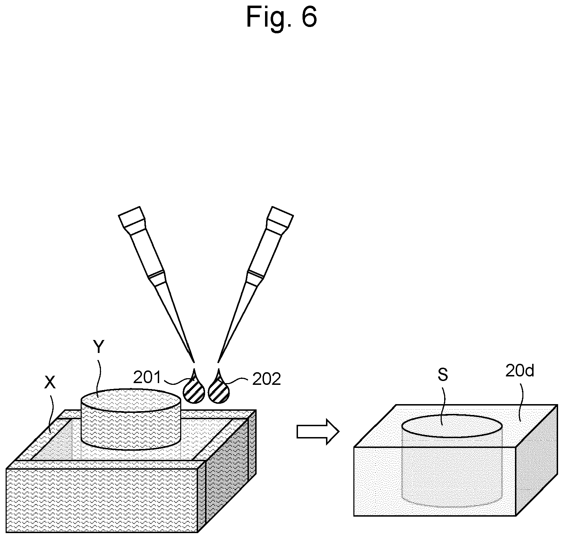

The layered body 1D can be prepared as follows. First, as illustrated in FIG. 6, a mold frame X is prepared according to the shape of the gel 20d covering the side. A core Y is placed in the mold frame X, and Liquid A 201 containing the multi-branched polymer A and Liquid B containing the multi-branched polymer B are added sequentially or simultaneously between the mold frame X and the core Y and allowed to gel to form the gel 20d. The gel 20d can be used like a culture container to form the cell layer 10a, the gel layer 20a, and the cell layer 10b in a cavity S of the gel 20d in the same manner as in the first embodiment to obtain the layered body 1D. The layered body 1D can be prepared without the use of the culture vessel 40 with a partition, as in the first embodiment. By containing a drug or a bio-derived liquid factor in the gel 20d covering the side, the above-described drug or the like can be released slowly from the gel 20d into the cell layers 10a and 10b.

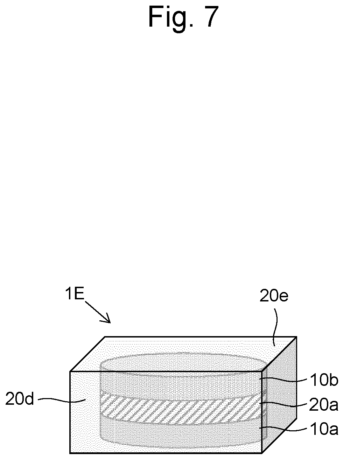

FIG. 7 is a schematic diagram illustrating a layered body 1E according to a fifth embodiment of the present invention. In a layered body 1E of FIG. 7, top of the layered body 1D of the fourth embodiment, the side of the layered body 1D being covered by a gel 20d containing a hydrogel, is further covered by a gel 20e containing a hydrogel. The hydrogel contained in the gel 20e is preferably the same hydrogel as the multi-branched polymer hydrogel contained in the gel layer 20a, but the hydrogel may be of a different type. The layered body 1E, like the layered body 1B in the second embodiment, can provide a barrier to the gel 20e at the top and protect the cell layer 10b. The advantages of the side being covered by the gel 20d are the same as in the case of the layered body 1D according to the fourth embodiment. The layered body 1E can be prepared, for example, by preparing the layered body 1D according to the fourth embodiment, and adding Liquid A containing a multi-branched polymer A and Liquid B containing a multi-branched polymer B on the top of the layered body 1D and allowing the mixture to gel to form a gel 20e.

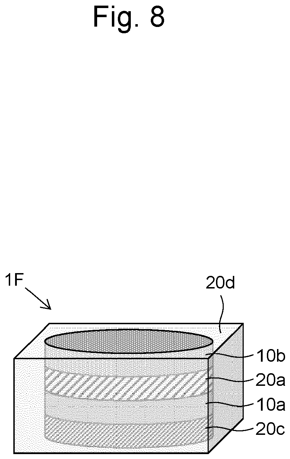

FIG. 8 is a schematic diagram illustrating a layered body 1F according to a sixth embodiment of the present invention. In the layered body 1F of FIG. 8, the side of the layered body 1C according to the third embodiment is further covered by a gel 20d containing a hydrogel. The hydrogel contained in the gel 20d is preferably the same hydrogel as the multi-branched polymer hydrogel contained in the gel layer 20A, but the hydrogel may be of a different type. As in the fourth embodiment, the layered body 1F can be obtained by forming a gel 20d corresponding to a side portion using a mold frame X and a core Y, and then using the gel 20d like a culture container to form a gel layer 20c, a cell layer 10a, a gel layer 20a, and a cell layer 10b in a cavity S of the gel 20d as in the third embodiment.

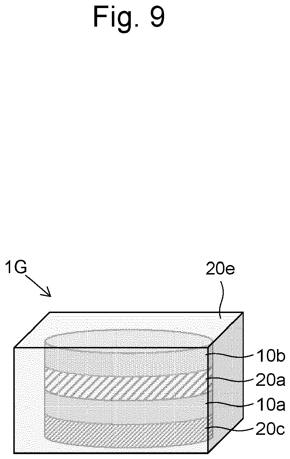

Next, a seventh embodiment of the present invention will be described based on FIG. 9. In a layered body 1G of FIG. 9, top of the layered body 1F of the sixth embodiment, the side of the layered body 1F being covered by a gel 20d containing a hydrogel, is further covered by a gel 20e containing a hydrogel. The hydrogel contained in the gel 20e is preferably the same hydrogel as the multi-branched polymer hydrogel contained in the gel layer 20a, but the hydrogel may be of a different type. In this layered body 1G, the entire circumference of the cell layers 10a and 10b is covered by a gel, and floating culture can be carried out in the state of the layered body 1G. The layered body 1G can be prepared, for example, by preparing the layered body 1F according to the sixth embodiment, and adding Liquid A containing a multi-branched polymer A and Liquid B containing a multi-branched polymer B on the top of the layered body 1F and allowing the mixture to gel to form a gel 20e.

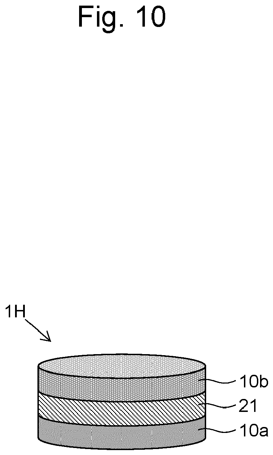

FIG. 10 illustrates a layered body 1H according to an eighth embodiment of the present invention. The layered body 1H of FIG. 10 has a layered structure in which a gel layer 21 containing a hydrogel is disposed between two cell layers 10a and 10b containing different types of cells from each other. In this embodiment, the gel layer 21 contains a drug or a bio-derived liquid factor. This allows the drug or the bio-derived liquid factor to be released slowly from the gel layer 21 to act on cells.

The agent or the bio-derived liquid factor that can be contained in the gel layer 21 is not limited, and can be appropriately selected in consideration of an effect on cells, details of a paracrine effect to be investigated, or the like, or two or more agents or bio-derived liquid factors may be contained in combination. Examples of the agent include dimethyl sulfoxide, Y27632, SB431542, dorsomorphine, CHIR99021, IWR-1, ascorbic acid, retinoic acid, forskolin, acetaminophen, 4-aminopyridine, a penicillin antibiotic (such as amoxicillin, ampicillin, or sultamicillin tosylate hydrate), a cephem antibiotic (such as cefcapenpovoxil hydrochloride, cefditoren pivoxil, or cefdinil), a macrolide antibiotic (clarithromycin, erythromycin, or azithromycin), a tetracycline (such as minocycline or doxycycline), a new quinolone (such as levofloxacin, tosfloxacin, or galenoxacin), and an amino acid (such as glutamic acid, methionine, glycine, phenylalanine, or arginine), and examples of the bio-derived liquid factor include NGF, BDNF, NT-3, TNF-α, HGF, IGF, VEGF, EGF, PDGF, TGF-β, IL-1, IL-2, IL-3, IL-4, IL-5, IL-6, IL-7, IL-8, IFN-α, IFN-β, and IFN-γ.

The layered body 1H can be prepared in accordance with the first embodiment, except that the gel layer 21 is formed by dissolving, suspending, or the like of a drug or a bio-derived liquid factor in at least one of Liquid A and Liquid B to form the gel layer 21.

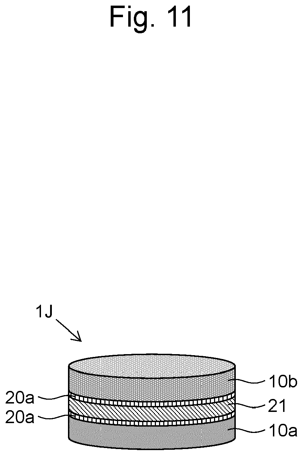

A ninth embodiment of the present invention will be described based on FIG. 11. The layered body 1J of FIG. 11 is formed in the same manner as the layered body 1H according to the eighth embodiment, except that the vicinity of the interface with the cell layers 10a and 10b of the gel layer 21 containing an agent or a bio-derived liquid factor is formed as a gel layer 20a which neither contains an agent nor a bio-derived liquid-derived factor in the layered body 1H. By placing a gel layer 20a which neither contains a drug nor a bio-derived liquid factor in the vicinity of the interface and sandwiching the gel layer 21 by the gel layers 20a, an effect of slow release of a drug or a bio-derived liquid factor from the gel layer 21 to the cell layers 10a and 10b can be delayed, and changes in the effect on cells over time can be examined in detail. The layered body 1J can be prepared in accordance with the first embodiment and the eighth embodiment, except that the gel layers 20a, 21, and 20a are formed sequentially by three steps.

The thickness of the layered body according to the first to ninth embodiments is not limited, and can be set to a suitable value to adequately detect a paracrine effect acting between the cell layers 10a and 10b, specifically, for example, preferably from 0.02 mm to 2 mm, and more preferably from 0.02 mm to 1 mm.

In the first to ninth embodiments, a case in which two cell layers are disposed has been described, but the number of cell layers may be three or more, and a paracrine effect acting between the three or more cell layers can be detected by a layered body in which a gel layer containing a hydrogel is disposed between the three or more cell layers. In this case, the types of cells in the three or more cell layers may all be different, and some (for example, two) of the three or more cell layers may contain cells of the same type.

Various tests can be carried out using the layered body of the present invention as described above. The layered body contains two or more different types of cells in separate cell layers rather than in a mixed state, and an effect on each type of cell when the layered body is stimulated can be detected in isolation. A paracrine effect of a protein secreted into a medium from a cell in one cell layer on cells in another cell layer can be detected with high intensity.

For example, the cell viability in a layered body can be measured by a method including the following steps.
(a) stimulating the layered body;
(b) staining the layered body with a staining reagent for measuring the cell viability;
(c) analyzing the stained layered body by image processing; and
(d) calculating the cell viability based on a result obtained by the analysis.

Here, the type of stimulus to a layered body is not particularly limited, and examples of stimuli can include various agents and environmental changes such as temperature. The type of staining reagent and the method of calculating the cell viability by image processing can be appropriately based on previously known methods. Since each cell layer is separated by an intervening gel layer, for example, when each cell layer is observed using a confocal microscope in step (c), cell layers in different positions in the z-axis direction can be observed, and information about living and dead cells in each cell layer can be acquired by image processing.

The expression level of at least one of RNA and protein can be evaluated using the layered body of the present invention. Specifically, the evaluation can be performed by going through the following steps.
(a) stimulating the layered body;
(b) collecting at least two cell layers separately; and
(c) measuring the expression level of at least one of RNA and protein for the collected cell layer.

Here, the measurement of at least one of RNA and protein expression levels can be performed by appropriately employing a conventional measurement method. Since each cell layer is separated by an intervening gel layer, for example, in step (c), a solution for collecting at least one of an RNA and a protein is added to a cell layer, the solution is collected, and then the gel layer is removed with a pipetman or the like, and the at least one of an RNA and a protein can be collected by the same step for another cell layer under the gel layer.

The cell viability in a layered body can also be measured by the following steps.
(a) stimulating the layered body;
(b) adding a reagent for measuring the cell viability to a culture medium; and
(c) collecting the culture medium to which the reagent is added, and measuring the cell viability.

Here, examples of the reagent for measuring the cell viability include MTT, WST-1, and WST-8. A cell layer on top of a gel layer is collected, including the gel layer, in a separate culture container, using a pipetman or the like, and a reagent for measuring the cell viability at the same time as a cell layer present on the bottom of the gel layer is added to a culture medium, and the cell viability can be calculated by measuring the absorbance of the culture medium.

Protein expression in a layered body can be evaluated by further going through the following steps.
(a) stimulating the layered body;
(b) adding a luminescent substrate to the layered body;
(c) measuring the luminescence intensity of the layered body; and
(d) evaluating expression of a protein based on a measurement result.

Here, the luminescent substrate may be selected from various conventionally known substrates, and examples of such substrates include D-luciferin and selenoterazine. Expression of a target protein in each cell layer can be measured based on the luminescence intensity by using cells into which a reporter gene is introduced for cells to be used in each cell layer, and detecting different luminescence wavelengths.

### EXAMPLES

The present invention is described below more concretely by way of Examples and Comparative Examples. However, the present invention is not limited to these Examples.

### (Example 1)

### · Preparation of Liquid A

In 2 mL of a phosphate buffered saline (manufactured by Life Technologies Corporation, hereinafter also referred to as PBS(-)), 0.04 g of Tetra-PEG-SH (trade name, SUNBRIGHT PTE-100SH; manufactured by Yuka Sangyo Co., Ltd.) was dissolved, and the resulting solution was filtered through a filter having an average pore size of 0.2 µm (trade name, Minisart Syringe Filter 175497K; manufactured by Sartorius), to prepare Liquid A in which the concentration of Tetra-PEG-SH was 2% by weight.

### · Preparation of Liquid B

In 5 mL of PBS(-), 0.1 g of Tetra-PEG-maleimide (trade name, SUNBRIGHT PTE-100MA; manufactured by Yuka Sangyo Co., Ltd.) was dissolved, and the resulting solution was filtered through a filter having an average pore size of 0.2 µm, to prepare Liquid B in which the concentration of Tetra-PEG-maleimide was 2% by weight.

### · Culture of Cells

In an incubator (trade name, KM-CC17RU2; manufactured by Panasonic Corporation; 37°C, environment of 5% by volume CO₂), HepG2 cells (JCRB Cell Bank, referred to as "HepG2"), and NIH/3T3 cells (JCRB Cell Bank, referred to as "3T3") were cultured for 72 hours in a 100 mm dish using Dulbecco's Modified Eagle's Medium (trade name, DMEM (1x); manufactured by Life Technologies Corporation; hereinafter referred to as "DMEM") supplemented with 10% calf serum (hereinafter also referred to as "serum").

### · Staining of Cells and Preparation of Cell Suspension

Green fluorescent dye (trade name, Cell Tracker Green; manufactured by Life Technologies) and orange fluorescent dye (trade name, Cell Tracker Orange, manufactured by Life Technologies Corporation) that were stored frozen was thawed and allowed to warm to room temperature (25°C). The dye was then dissolved at a concentration of 10 mmol/L (mM) in dimethyl sulfoxide (hereinafter referred to as "DMSO"). The resulting solution was mixed with serum-free DMEM to prepare serum-free DMEM containing the green fluorescent dye and serum-free DMEM containing the orange fluorescent dye at a concentration of 10 µmol/L (µM). 5 mL of the serum-free DMEM containing the green fluorescent dye was added to a dish containing the cultured HepG2, 5 mL of the serum-free DMEM containing the orange fluorescent dye was added to a dish containing the cultured 3T3, followed by staining in an incubator for 30 minutes. Thereafter, the supernatant was removed using an aspirator. To the dishes, 5 mL of PBS(-) was added, and then the PBS(-) was removed by aspiration using an aspirator, to wash the surface. After performing two times of the washing operation using PBS(-), 2 mL of 0.05% trypsin-0.05% EDTA solution (manufactured by Life Technologies Corporation) was added to the dishes, and the dishes were then incubated in an incubator for 5 minutes to detach cells from the dishes. After confirmation of the detachment of the cells under a phase contrast microscope (apparatus name, CKX41; manufactured by Olympus Corporation), 4 mL of DMEM supplemented with serum was added to each dish to deactivate the trypsin. The cell suspensions in the dishes were combined and transferred into one 15-mL centrifuge tube, and centrifugation (trade name, H-19FM; manufactured by KOKUSAN Co., Ltd.; 1.2 × 10³ rpm, 5 minutes, 5°C) was carried out, followed by removal of the supernatant using an aspirator. Thereafter, 2 mL of DMEM supplemented with serum was added to the centrifuge tube, and gentle pipetting was carried out to disperse the cells, to obtain a cell suspension. From the cell suspension, a 20-µL aliquot was taken into an Eppendorf tube, and 20 µL of 0.4% trypan blue staining solution was added to the tube, followed by pipetting. From the stained cell suspension, a 20-µL aliquot was taken and placed on a PMMA plastic slide. The number of cells was counted using a Countess (trade name, Countess Automated Cell Counter; manufactured by Life Technologies Corporation), to determine the number of cells in the suspension.

### · Preparation of Extracellular Substrate Solution

Matrigel (manufactured by Corning Inc., registered trademark) was mixed well with serum-free DMEM at 1:1 and stored at 4°C until immediately before use.

### · Preparation of Layered Body

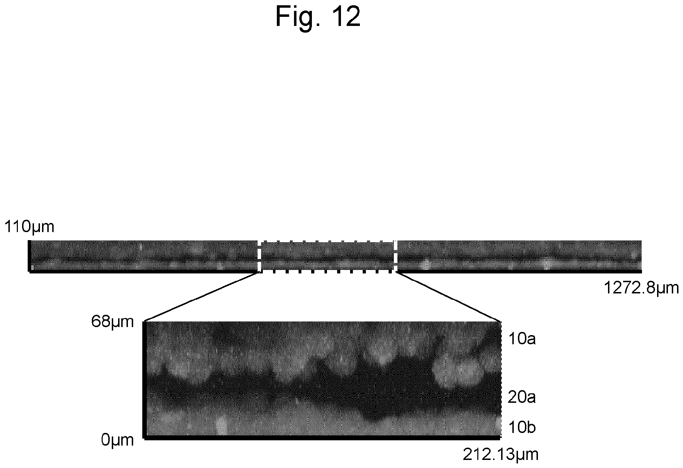

A flexiPERM (registered trademark) micro12 reusable (manufactured by SARSTEDT Inc.) was attached to a cover glass, and 2 × 10⁴ cells of HepG2 suspended in DMEM with serum stained with the green fluorescent dye described above were seeded and incubated in an incubator for at least 16 hours. After the incubation, the culture medium was removed with a pipetman, and 16 µl of Liquid A, which was prepared immediately before use, was added to the culture medium, and Liquid B was added and allowed to gel. After confirming gelation, 50 µl of an extracellular substrate solution was added and incubated at 37°C for 30 minutes. After the incubation, the extracellular substrate solution was removed, and 2 × 10⁴ cells of 3T3 suspended in DMEM with serum stained with the orange fluorescent dye were seeded and incubated in an incubator for 24 hours. After the incubation, a layered body was observed under a confocal microscope (FV10i, manufactured by OLYMPUS Corporation), and a three-layered structure including a cell layer 10a, a gel layer 20a, and a cell layer 10b was confirmed (FIG. 12).

### (Example 2 and Comparative Example 1)

### · Preparation of Liquid A

In 2 mL of a phosphate buffered saline (manufactured by Life Technologies Corporation, hereinafter also referred to as PBS(-)), 0.04 g of Tetra-PEG-SH (trade name, SUNBRIGHT PTE-100SH; manufactured by Yuka Sangyo Co., Ltd.) was dissolved, and the resulting solution was filtered through a filter having an average pore size of 0.2 µm (trade name, Minisart Syringe Filter 175497K; manufactured by Sartorius), to prepare Liquid A in which the concentration of Tetra-PEG-SH was 2% by weight.

### · Preparation of Liquid B

In 5 mL of PBS(-), 0.1 g of Tetra-PEG-maleimide (trade name, SUNBRIGHT PTE-100MA; manufactured by Yuka Sangyo Co., Ltd.) was dissolved, and the resulting solution was filtered through a filter having an average pore size of 0.2 µm, to prepare Liquid B in which the concentration of Tetra-PEG-maleimide was 2% by weight.

### · Culture of Cells

In an incubator (trade name, KM-CC17RU2; manufactured by Panasonic Corporation; 37°C, environment of 5% by volume CO₂), HepG2 cells and LI90 cells (JCRB Cell Bank, hereinafter referred to as "LI90") were cultured for 72 hours in a 100 mm dish using Dulbecco's Modified Eagle's Medium (trade name, DMEM (1x); manufactured by Life Technologies Corporation; hereinafter referred to as "DMEM") supplemented with 10% fetal bovine serum (hereinafter also referred to as "FBS").

### · Preparation of Cell Suspension

To the dishes, 5 mL of PBS(-) was added, and then the PBS(-) was removed by aspiration using an aspirator, to wash the surface. After performing two times of the washing operation using PBS(-), 2 mL of 0.05% trypsin-0.05% EDTA solution (manufactured by Life Technologies Corporation) was added to the dishes, and the dishes were then incubated in an incubator for 5 minutes to detach cells from the dishes. After confirmation of the detachment of the cells under a phase contrast microscope (apparatus name, CKX41; manufactured by Olympus Corporation), 4 mL of DMEM supplemented with FBS was added to each dish to deactivate the trypsin. The cell suspensions in the dishes were combined and transferred into one 15-mL centrifuge tube, and centrifugation (trade name, H-19FM; manufactured by KOKUSAN Co., Ltd.; 1.2 × 10³ rpm, 5 minutes, 5°C) was carried out, followed by removal of the supernatant using an aspirator. Thereafter, 2 mL of DMEM supplemented with FBS was added to the centrifuge tube, and gentle pipetting was carried out to disperse the cells, to obtain a cell suspension. From the cell suspension, a 20-µL aliquot was taken into an Eppendorf tube, and 20 µL of 0.4% trypan blue staining solution was added to the tube, followed by pipetting. From the stained cell suspension, a 20-µL aliquot was taken and placed on a PMMA plastic slide. The number of cells was counted using a Countess (trade name, Countess Automated Cell Counter; manufactured by Life Technologies Corporation), to determine the number of cells in the suspension as in Example 1.

### · Preparation of Layered Body

5 × 10⁴ cells of the LI90 suspended in DMEM with serum were seeded in a 24-well multiplate and incubated in an incubator for at least 16 hours (cell layer A). After the incubation, the culture medium was removed with a pipetman, and 16 µl of Liquid A, which was prepared immediately before use, was added to the culture medium, and Liquid B was added and allowed to gel (gel layer A). After confirming gelation, 50 µl of an extracellular substrate solution was added and incubated at 37°C for 30 minutes. After the incubation, the extracellular substrate solution was removed, and 5 × 10⁴ cells of HepG2 suspended in DMEM with serum were seeded and incubated in an incubator for 24 hours to form a cell layer B to prepare a layered body (Example 2).

### · Cell Seeding in Insert

In a 24-well multiplate, 5 × 10⁴ cells of the LI90 suspended in DMEM with serum were seeded, and in an insert (manufactured by Corning), 5 × 10⁴ cells of the HepG2 suspended in DMEM with serum were seeded. The cells were then incubated in an incubator for at least 16 hours (Comparative Example 1).

### · Comparative Experiment

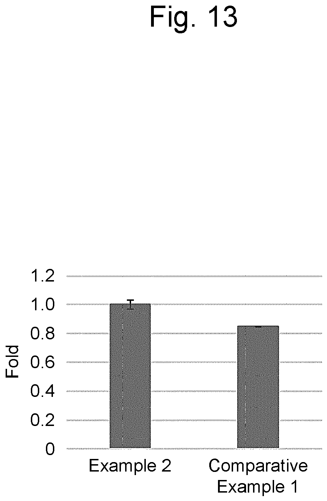

To the layered body of Example 2 prepared as described above and HepG2 of the insert in Comparative Example 1, ethanol (manufactured by Wako Pure Chemical Corporation) to a final concentration of 1 mM and acetaminophen (manufactured by Tokyo Chemical Industry Co., Ltd.) to a final concentration of 15 mM were added and incubated for 24 hours. After removal of the culture medium and washing with PBS(-), messenger RNA (hereinafter referred to as "mRNA") was extracted with RNAeasy (manufactured by QIAGEN) according to the procedure, and complementary DNA (hereinafter referred to as "cDNA") was synthesized with a SuperScript Kit (manufactured by Thermo Fisher Scientific Inc.). The expression levels of the type I collagen α1 chain were compared by quantitative PCR (hereinafter referred to as "qPCR") using the synthesized cDNA using the TaqMan probe (manufactured by Thermo Fisher Scientific Inc.). The results are shown in FIG. 13. The results in FIG. 13 indicated that the RNA expression of cells in the layered body of the present invention could be evaluated.

### (Example 3 and Comparative Example 2)

### · Preparation of Liquid A

In 2 mL of a phosphate buffered saline (manufactured by Life Technologies Corporation, hereinafter also referred to as PBS(-)), 0.04 g of Tetra-PEG-SH (trade name, SUNBRIGHT PTE-100SH; manufactured by Yuka Sangyo Co., Ltd.) was dissolved, and the resulting solution was filtered through a filter having an average pore size of 0.2 µm (trade name, Minisart Syringe Filter 175497K; manufactured by Sartorius), to prepare Liquid A in which the concentration of Tetra-PEG-SH was 2% by weight.

### · Preparation of Liquid B

In 5 mL of PBS(-), 0.1 g of Tetra-PEG-maleimide (trade name, SUNBRIGHT PTE-100MA; manufactured by Yuka Sangyo Co., Ltd.) was dissolved, and the resulting solution was filtered through a filter having an average pore size of 0.2 µm, to prepare Liquid B in which the concentration of Tetra-PEG-maleimide was 2% by weight.

### · Culture of Cells

In an incubator (trade name, KM-CC17RU2; manufactured by Panasonic Corporation; 37°C, environment of 5% by volume CO₂), SH-SY5Y cells (ECACC, hereinafter referred to as "SH-SY5Y") and U251MG cells (ECACC, hereinafter referred to as "U251MG") and human umbilical cord epithelial cells (LONZA, hereinafter referred to as "HUVEC") were cultured in 100 mm dishes for 72 hours. The culture medium for each cell was culture medium for SH-SY5Y (manufactured by Dainippon Sumitomo Pharma Co., Ltd.), No. 105 medium (manufactured by Dainippon Sumitomo Pharma Co., Ltd.) for U251MG, and EGM-2 (manufactured by LONZA) for HUVEC.

### · Preparation of Extracellular Substrate Solution

Matrigel (manufactured by Corning Inc., registered trademark) was mixed well with serum-free DMEM at 1:1 and stored at 4°C until immediately before use.

### · Preparation of Layered Body

In a 96-well multiplate (manufactured by Corning Inc.), 2 × 10⁴ cells of SH-SY5Y were seeded and incubated in an incubator for at least 16 hours. After the incubation, the culture medium was removed with a pipetman, and 16 µl of Liquid A, which was prepared immediately before use, was added to the culture medium, and Liquid B was added and allowed to gel. After confirming gelation, 50 µl of an extracellular substrate solution was added and incubated at 37°C for 30 minutes. After the incubation, the extracellular substrate solution was removed, and 2 × 10⁴ cells of U251MG were seeded and incubated in an incubator for 2 hours. After confirming that the cells were adhering, the culture medium was removed with a pipetman, 16 µl of Liquid A prepared immediately before use was added, and Liquid B was added and allowed to gel. After confirming gelation, 50 µl of the extracellular substrate solution was added and incubated at 37°C for 30 minutes. After the incubation, the extracellular substrate solution was removed and 2 × 10⁴ cells of HUVEC were seeded and incubated in an incubator for 24 hours to prepare a layered body (Example 3). A layered body with three cell layers formed only with SH-SY5Y was also prepared (Comparative Example 2). The preparation method was the same as described above.

### · Preparation of Viability Measurement Reagent

A frozen WST-1 reagent (manufactured by DOJINDO LABORATORIES) was brought to room temperature and diluted 10 times in EGM-2.

### · Comparative Experiment of Co-culture

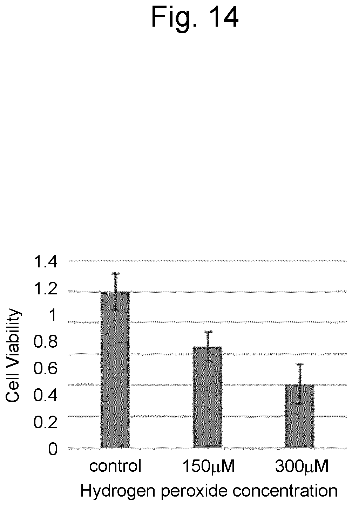

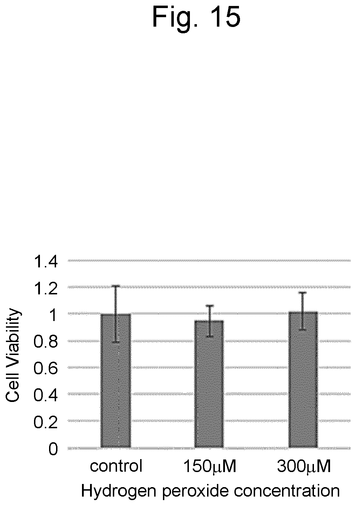

Hydrogen peroxide (Wako Pure Chemical Corporation) was added to the layered body of Example 3 and Example 2 at final concentrations to expose the layered body to 0, 150, and 300 µM, adjusted with PBS (-) and incubated for 24 hours. After the incubation, the culture medium containing hydrogen peroxide water was removed, 100 µl of the viability measurement reagent was added and incubated at 37°C for 2 hours. After the incubation, the culture medium was collected from each well and the absorbance was measured with a plate reader (Cytation, manufactured by Biotech Co., Ltd.). The measurement results are illustrated in FIG. 14 and FIG. 15. The layered body prepared with three cell layers of different types of cells (FIG. 15) indicated significantly higher viability (p < 0.05) at 150 and 300 µM exposure to hydrogen peroxide than the layered body prepared with three cell layers of the same cells (FIG. 14). The reason for this may be due to the transmission of a protective humoral factor from 3T3 to HepG2 by paracrine.

### (Reference Example 1)

### · Preparation of Liquid A

In 2 mL of a phosphate buffered saline (manufactured by Life Technologies Corporation, hereinafter also referred to as PBS(-)), 0.04 g of Tetra-PEG-SH (trade name, SUNBRIGHT PTE-100SH; manufactured by Yuka Sangyo Co., Ltd.) was dissolved, and the resulting solution was filtered through a filter having an average pore size of 0.2 µm (trade name, Minisart Syringe Filter 175497K; manufactured by Sartorius), to prepare Liquid A in which the concentration of Tetra-PEG-SH was 2% by weight.

### · Preparation of Liquid B

In 5 mL of PBS(-), 0.1 g of Tetra-PEG-maleimide (trade name, SUNBRIGHT PTE-100MA; manufactured by Yuka Sangyo Co., Ltd.) was dissolved, and the resulting solution was filtered through a filter having an average pore size of 0.2 µm, to prepare Liquid B in which the concentration of Tetra-PEG-maleimide was 2% by weight.

### · Preparation of Gel Layer

Liquid A was added in an insert, and then, Liquid B was added in the insert. The amount of Liquid A and the amount of Liquid B to be added was the same, and Liquid A and Liquid B were added so that the gel layer was 1, 2, and 4 mm thick.

### · Preparation of Fluorescence-labeled Molecular Weight Standard Solution

Dextrans (manufactured by Thermo Fisher Scientific Inc.) with molecular weights of 570 Da, 10 kDa, and 500 kDa were diluted to 1% by weight in PBS (-).

### · Examination of Permeability

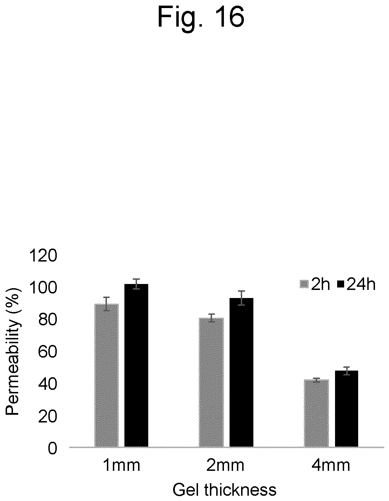

600 µl of PBS (-) was added in a 24-well multiplate, and the insert in which the gel layer was prepared to which 300 µl of the fluorescence-labeled molecular weight standard was added was set in the 24-well multiplate. After incubation in an incubator for 2 or 24 hours, PBS (-) at the bottom of the insert was collected, and the fluorescence intensity was measured by a plate reader (Cytation, manufactured by Biotech Co., Ltd.) for absorbance. The measurement results are illustrated in FIG. 16. As is clear from FIG. 16, even with gel layers having thicknesses of 1, 2, and 4 mm, permeation and penetration of the gel layers was achieved in 2 and 24 hours.

### (Reference Example 2)

### · Preparation of Liquid A

In a predetermined amount of a phosphate buffered saline (manufactured by Life Technologies Corporation, hereinafter also referred to as PBS(-)), Tetra-PEG-SH (trade name, SUNBRIGHT PTE-100SH; manufactured by Yuka Sangyo Co., Ltd.) was dissolved, and the resulting solution was filtered through a filter having an average pore size of 0.2 µm (trade name, Minisart Syringe Filter 175497K; manufactured by Sartorius), to prepare Liquid A in which the concentration of Tetra-PEG-SH was 0.7, 1, 2, 4, and 8% by weight.

### · Preparation of Liquid B

In PBS(-), a predetermined amount of Tetra-PEG-maleimide (trade name, SUNBRIGHT PTE-100MA; manufactured by Yuka Sangyo Co., Ltd.) was dissolved, and the resulting solution was filtered through a filter having an average pore size of 0.2 µm, to prepare Liquid B in which the concentration of Tetra-PEG-maleimide was 0.7, 1, 2, 4, and 8% by weight.

### · Concentration of Gel Layer

50 µl of Liquid A was dropped into a 96-well multiplate, and the same amount of Liquid B was dropped into the 96-well multiplate. After confirming the gelation, the surface roughness of the gel layer was checked under a microscope. The results are illustrated in Table 1. In Table 1, "Good" indicates that no unevenness was observed in a phase-contrast microscope. As indicated in Table 1, no unevenness was observed at all concentrations at a level where a shadow could be seen on the surface by the phase-contrast microscopy.

**[Table 1]**

| Table 1. Surface roughness measurement result | | | | | |
|---|---|---|---|---|---|
| Concentration (weight %) | 0.7 | 1 | 2 | 4 | 8 |
| Determination | Good | Good | Good | Good | Good |

### (Reference Example 3)

### · Preparation of Liquid A

In a predetermined amount of a phosphate buffered saline (manufactured by Life Technologies Corporation, hereinafter also referred to as PBS(-)), Tetra-PEG-SH (trade name, SUNBRIGHT PTE-100SH; manufactured by Yuka Sangyo Co., Ltd.) was dissolved, and the resulting solution was filtered through a filter having an average pore size of 0.2 µm (trade name, Minisart Syringe Filter 175497K; manufactured by Sartorius), to prepare Liquid A in which the concentration of Tetra-PEG-SH was 2% by weight.

### · Preparation of Liquid B

In PBS(-), a predetermined amount of Tetra-PEG-maleimide (trade name, SUNBRIGHT PTE-100MA; manufactured by Yuka Sangyo Co., Ltd.) was dissolved, and the resulting solution was filtered through a filter having an average pore size of 0.2 µm, to prepare Liquid B in which the concentration of Tetra-PEG-maleimide was 2% by weight.

### · Preparation of Gel Layer

50 µl of Liquid A was dropped onto a glass substrate, and the same amount of Liquid B was dropped onto the glass substrate. After confirming the gelation, the thickness of the gel was measured by confocal measurement. As a result, the thickness of the gel layer was 20 um.

### (Example 4)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 2% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 2% by weight.

### · Culture of Cells

3T3 and 3T3-luciferase cells (ECACC, hereinafter referred to as "3T3-luc") were cultured in 100 mm dishes in an incubator for 72 hours. The culture medium for each cell was DMEM for both 3T3 and 3T3-luc.

### · Preparation of Layered Body

A layered was prepared in the same procedure as in Example 2. For the cell layer A, 3T3-luc was used, and for the cell layer B, 3T3 was used. In order to examine the gel barrier properties, an additional gel layer B was prepared on top of the cell layer B. A layered body without the gel layer B was prepared as a control.

### · Preparation of Cell Reaction Solution

TNF-α (manufactured by Wako Pure Chemical Corporation) was diluted with distilled water. D-luciferin (manufactured by Wako Pure Chemical Corporation) was diluted with Tris buffer at pH 7.8.

### Comparative Experiment of Gel Barrier Properties

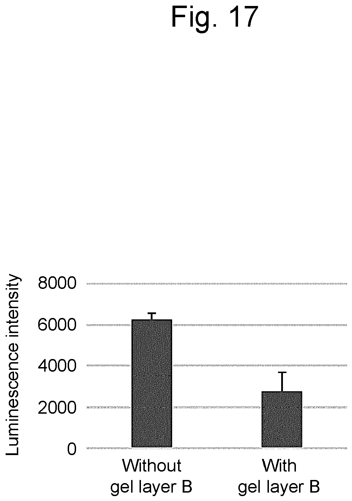

TNF-α was added to the layered body to a final concentration of 50 ng/ml, and incubated at 37°C for 3 hours. After three hours, D-luciferin was added to the layered body to a final concentration of 200 µM, incubated at 37°C for 5 minutes, and the luminescence intensity was measured with a plate reader. The measurement results are illustrated in FIG. 17. As is clear from the results in FIG. 17, when the gel layer B was prepared, the luminescence intensity of the cells was significantly lower than the luminescence intensity of the control (p < 0.05).

### (Reference Example 4)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 4% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 4% by weight.

### · Preparation of Gel Layer

Liquid A was added in an insert, and then, Liquid B was added in the insert. The amount of Liquid A and the amount of Liquid B to be added was the same, and Liquid A and Liquid B were added so that the gel layer was 1, 2, and 4 mm thick.

### · Preparation of Fluorescence-labeled Molecular Weight Standard Solution

Dextrans with molecular weights of 500 kDa were diluted to 1% by weight in PBS (-).

### · Experiment for Examining Gel Barrier Properties

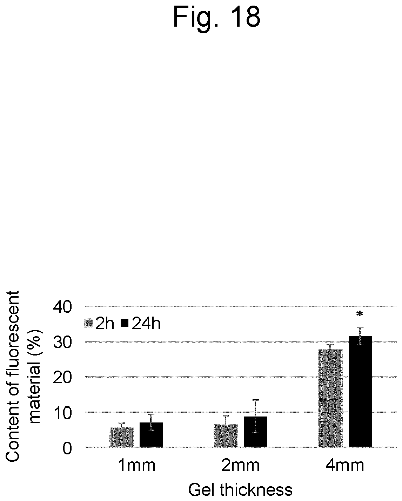

600 µl of PBS(-) was added to a 24-well multiplate, and the insert in which the gel layer was prepared to which 300 µl of the fluorescence-labeled molecular weight standard solution was added was set in the 24-well multiplate. After incubation in an incubator for 2 and 24 hours, the gel layer was collected, and the fluorescence intensity of the gel was measured using a plate reader. The measurement results are shown in FIG. 18. The fluorescence intensity was significantly higher at 24 hours than at 2 hours at a thickness of 4 mm (p < 0.05). The reason for this is assumed to be due to the time-dependent capture of dextran in the network structure of a hydrogel.

### (Examples 5 and 6)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 2% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 2% by weight.

### · Culture of Cells

SH-SY5Y cells (ECACC, hereinafter referred to as "SH-SY5Y") and U251MG cells (ECACC, hereinafter referred to as "U251MG"), and human umbilical cord epithelial cells (LONZA, hereinafter referred to as "HUVECs") were cultured in 100 mm dishes in an incubator for 72 hours. The culture medium for each cell was medium for SH-SY5Y (Dainippon Sumitomo Pharma Co., Ltd.) for SH-SY5Y, No. 105 medium (Dainippon Sumitomo Pharma Co., Ltd.) for U251MG, and EGM-2 (manufactured by LONZA Co., Ltd.) for HUVEC. Subsequently, a cell suspension was prepared in the same manner as in Example 1.

### · Preparation of Extracellular Substrate Solution

Matrigel (manufactured by Corning Inc., registered trademark) was mixed well with serum-free DMEM at 1:1 and stored at 4°C until immediately before use.

### · Preparation of Layered Body

For preparing a layered body (Example 5) with an additional gel layer at the bottom of the cell layer A, flexiPERM (registered trademark) micro12 reusable was attached to a cover glass, 16 µl of Liquid A was added to wells, followed by an equal amount of Liquid B to prepare a gel layer A, and 50 µl of the extracellular substrate solution was added and incubated at 37°C for 30 minutes. After the incubation, the extracellular substrate solution was removed, and 2 × 10⁴ cells of SH-SY5Y were seeded and incubated in an incubator for 2 hours. After the incubation, the culture medium was removed with a pipetman, 16 µl of Liquid A was added and an equal amount of Liquid B was added and allowed to gel. After confirming gelation, 50 µl of the extracellular substrate solution was added and incubated at 37°C for 30 minutes. After the incubation, the extracellular substrate solution was removed, 2 × 10⁴ cells of U251MG were seeded and incubated in an incubator for 2 hours. After the incubation, the culture medium was removed with a pipetman, 16 µl of Liquid A was added and an equal amount of Liquid B was added and allowed to gel. After confirming gelation, 50 µl of the extracellular substrate solution was added and incubated at 37°C for 30 minutes. After the incubation, the extracellular substrate solution was removed and 2 × 104cells of HUVEC were seeded and incubated in an incubator for 24 hours to prepare the layered body of Example 5. On the other hand, a layered body (Example 6) without a gel layer at the bottom was prepared in the same manner as in Example 1.

### · Collection Steps and Required Time Comparison

A scalpel (manufactured by Kai Corporation) was used to separate flexiPERM (registered trademark) micro12 reusable from the side face of a layered body, and the layered body was left in contact with only the side face of the cover glass. The layered body of Example 5 which is in contact with the cover glass by a gel layer was separated from the cover glass with a scalpel in the same manner as the side face. The layered body of Example 6 which is in contact with the cover glass by a cell layer was placed in a 35 mm dish with the cover glass, 5 mL of PBS (-) was added to the dish, the PBS (-) was aspirated off with an aspirator, and the surface was washed. After repeating the washing process with PBS (-) twice, 0.5 mL of 0.05% trypsin-0.05% EDTA solution was added to the dish, and heated in an incubator for 5 minutes to detach the layered body from the dish. The time required for detachment was measured. The results are indicated in Table 2. In Table 2, "Very good" means that the cells were detached without any remaining cells, and "Good" means that 30% of the cells remained. As shown in Table 2, when the gel was placed at the bottom layer, the time required for detachment was shorter.

**[Table 2]**

| Table 2. Measurement results of time required for detachment | | |
|---|---|---|
| | Gel layer at bottom layer | Cell layer at bottom layer |
| Time required for detachment (min) | 1 | 7 |
| Determination | Very good | Good |

### (Example 7)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 2% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 2% by weight.

### · Preparation of Layered Body Support Substrate Using Hydrogel

A mold for forming a hydrogel layered body support substrate was prepared using dimethylpolysiloxane (manufactured by Dow Chemical Company, hereinafter referred to as "PDMS"). The mold prepared was filled with 75 µl of Liquid A, and then an equal amount of Liquid B was added and allowed to gel. After confirmation of gelation, the hydrogel layered body support substrate was detached from the mold made with PDMS. The substrate was stored in PBS(-) in water until immediately before use.

### · Culture and Staining of Cells and Preparation of Cell Suspensions

Cells were cultured and stained in the same manner as in Example 1, and cell suspensions were prepared.

### · Preparation of Extracellular Substrate Solution

Matrigel (manufactured by Corning Inc., registered trademark) was mixed well with serum-free DMEM at 1:1 and stored at 4°C until immediately before use.

### · Preparation of Layered Body

The hydrogel layered body support substrate was placed on a cover glass, and 2 × 10⁴ cells of HepG2 suspended in DMEM with serum stained with green fluorescent dye were seeded and incubated in an incubator for at least 16 hours. After the incubation, the culture medium was removed with a pipetman, 16 µl of Liquid A prepared immediately before use was added and an equal amount of Liquid B was added and allowed to gel. After confirming gelation, 50 µl of the extracellular substrate solution was added and incubated at 37°C for 30 minutes. After the incubation, the extracellular substrate solution was removed, 2 × 10⁴ cells of 3T3 suspended in DMEM with serum stained with orange fluorescent dye were seeded and incubated in an incubator for 24 hours.

### · Observation of Layered Body

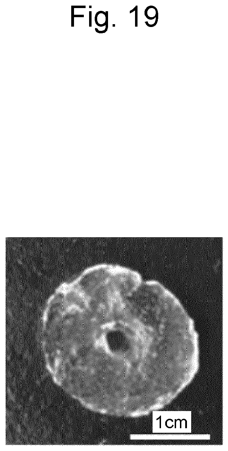

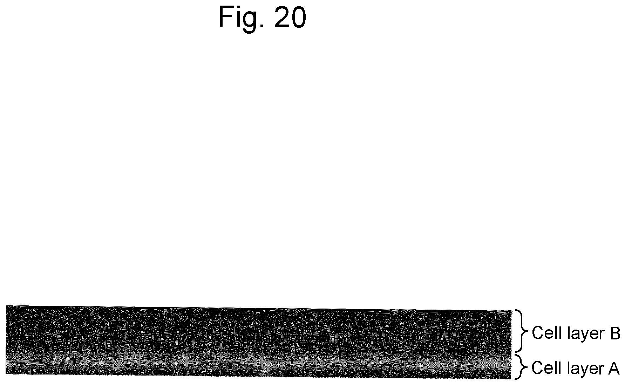

The layered body was observed with a confocal microscope. As shown in FIG. 19 and FIG. 20, it was confirmed that the internal structure of the layered body was prepared even when the side face of the layered body was not in contact with the culture container.

### (Examples 8 and 9)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 2% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 2% by weight.

### · Preparation of Layered Body Support Substrate Using Hydrogel

A layered body support substrate made of hydrogel was prepared in the same manner as in Example 7.

### · Culture and Staining of Cells and Preparation of Cell Suspensions

Cells were cultured and stained in the same manner as in Example 1, and cell suspensions were prepared.

### · Preparation of Extracellular Substrate Solution

Matrigel (manufactured by Corning Inc., registered trademark) was mixed well with serum-free DMEM at 1:1 and stored at 4°C until immediately before use.

### · Preparation of Layered Body

The hydrogel layered body support substrate was placed on a cover glass, and 2 × 10⁴ cells of HepG2 suspended in DMEM with serum stained with green fluorescent dye were seeded and incubated in an incubator for at least 16 hours. After the incubation, the culture medium was removed with a pipetman, 16 µl of Liquid A prepared immediately before use was added and an equal amount of Liquid B was added and allowed to gel. After confirming gelation, 50 µl of the extracellular substrate solution was added and incubated at 37°C for 30 minutes. After the incubation, the extracellular substrate solution was removed, and 2 × 10⁴ cells of 3T3 suspended in DMEM with serum stained with orange fluorescent dye were seeded. Incubation was carried out in an incubator for 24 hours to prepare a layered body of Example 8.

A layered body of Example 9, in which the top of the cell layer was covered entirely with the hydrogel, was prepared by seeding 2 × 104 cells of 3T3 suspended in DMEM with serum stained with orange fluorescent dye in the process of preparing the layered body of Example 8, then incubating the cells at 37°C for 2 hours, then removing the culture medium, adding 16 µl of the Liquid A, and covering the top of the cell layer with an equal amount of the Liquid B.

### · Examination of drying of layered bodies

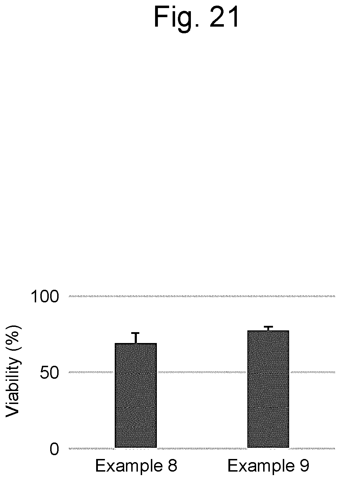

The layered body of Example 8, and the layered body of Example 9 in which the top of the cell layer was covered with hydrogel were taken out of the medium and allowed to stand in the room for 30 minutes. In order to see the effect of drying on the cell viability, propidium iodide (manufactured by Sigma Aldrich, hereinafter referred to as "PI") was diluted to 0.5 ng/ml in a medium, and each layered body was immersed in a PI solution and incubated at 37°C for 60 minutes. The layered body was observed under a confocal microscope, and the viability was calculated. The measurement results are illustrated in FIG. 21. As can be seen from FIG. 21, the layered body of Example 9, in which the top of the cell layer was covered with hydrogel, had a significantly higher survival rate than the layered body of Example 8, in which the top of the cell layer was not covered (p < 0.05).

### (Examples 10 and 11)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 2% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 2% by weight.

### · Preparation of Layered Body Support Substrate Using Hydrogel

A layered body support substrate made of hydrogel was prepared in the same manner as in Example 7.

### · Culture and Staining of Cells and Preparation of Cell Suspensions

Cells were cultured and stained in the same manner as in Example 1, and cell suspensions were prepared.

### · Preparation of Extracellular Substrate Solution

Matrigel (manufactured by Corning Inc., registered trademark) was mixed well with serum-free DMEM at 1:1 and stored at 4°C until immediately before use.

### · Preparation of Layered Body

The hydrogel layered body support substrate was placed on a cover glass, and 2 × 10⁴ cells of HepG2 suspended in DMEM with serum stained with green fluorescent dye were seeded and incubated in an incubator for at least 16 hours. After the incubation, the culture medium was removed with a pipetman, 16 µl of Liquid A prepared immediately before use was added and an equal amount of Liquid B was added and allowed to gel. After confirming gelation, 50 µl of the extracellular substrate solution was added and incubated at 37°C for 30 minutes. After the incubation, the extracellular substrate solution was removed, 2 × 10⁴ cells of 3T3 suspended in DMEM with serum stained with orange fluorescent dye were seeded and incubated in an incubator for 24 hours, and a layered body of Example 10 was prepared.

A layered body of Example 11, in which a gel layer is in contact with a culture container, was prepared as follows. The hydrogel layered body support substrate was placed on a cover glass, 16 µl of the Liquid A was added and an equal amount of Liquid B was added and allowed to gel, then 50 µl of an extracellular substrate was added and incubated at 37°C for 30 minutes, and the extracellular substrate was removed. 2 × 10⁴ cells of HepG2 suspended in DMEM with serum stained with the green fluorescent dye were seeded and incubated, then the culture medium was removed, 16 µl of the Liquid A was added, an equal amount of Liquid B was added and allowed to gel. 50 µl of the extracellular substrate was added and incubated at 37°C for 30 minutes, and the extracellular substrate was removed. 2 × 10⁴ cells of 3T3 suspended in DMEM with serum stained with orange fluorescent dye were seeded and incubated in an incubator for 24 hours to prepare a layered body of Example 11.

### · Confirmation of Layered Structure

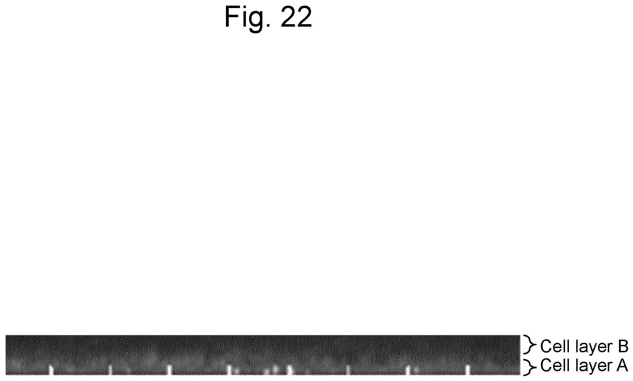

The layered body of Example 10 prepared was collected on a cover glass for observation and observed by confocal microscope to see if a layered structure was maintained (FIG. 22). From FIG. 22, it was confirmed that the layered structure was not broken.

### · Preparation of Reagents for Viability Measurement and Staining

Hoechst 33342 (manufactured by Thermo Fisher Scientific Inc.) was added to the layered body of Example 10 to a final concentration of 5 µg/ml and propidium iodide (Sigma Aldrich) was added to the layered body of Example 10 to a final concentration of 0.5 ng/ml and incubated at 37°C for 30 minutes.

### · Measurement of Viability

Images were captured using confocal microscopy and image analysis was performed to calculate the viability. The results are indicated in Table 3. In Table 3, "Good" means that the viability was 75%. The calculated viability was 82%.

**[Table 3]**

| Table 3. Viability Measurement Result | |
|---|---|
| Viability | 82% |
| Determination | Good |

### (Example 12)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 2% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 2% by weight.

### · Preparation of layered body support substrate using hydrogel

A layered body support substrate made of hydrogel was prepared in the same manner as in Example 7.

### · Culture of Cells and Preparation of Cell Suspensions

Cells were cultured and stained in the same manner as in Example 1, and cell suspensions were prepared.

### · Preparation of Extracellular Substrate Solution

Matrigel (manufactured by Corning Inc., registered trademark) was mixed well with serum-free DMEM at 1:1 and stored at 4°C until immediately before use.

### · Preparation of Layered Body

A layered body in which a gel layer is in contact with a culture container was prepared as follows. The hydrogel layered body support substrate was placed on a cover glass, 16 µl of the Liquid A was added and an equal amount of Liquid B was added and allowed to gel, then 50 µl of an extracellular substrate was added and incubated at 37°C for 30 minutes, and the extracellular substrate was removed. 2 × 10⁴ cells of 3T3 suspended in DMEM with serum were seeded and incubated, then the culture medium was removed, 16 µl of the Liquid A was added, an equal amount of Liquid B was added and allowed to gel. 50 µl of the extracellular substrate was added and incubated at 37°C for 30 minutes, and the extracellular substrate was removed. 2 × 10⁴ cells of 3T3 suspended in DMEM with serum were seeded and incubated in an incubator for one week to prepare a layered body.

### · Preparation of Reagents for Viability Measurement and Staining

Hoechst 33342 (manufactured by Thermo Fisher Scientific Inc.) was added to the layered body of Example 10 to a final concentration of 5 µg/ml and propidium iodide (Sigma Aldrich) was added to the layered body of Example 10 to a final concentration of 0.5 ng/ml and incubated at 37°C for 30 minutes.

### · Measurement of Viability

Images were captured using confocal microscopy and image analysis was performed to calculate the viability. The results was 77% (Table 4). In Table 4, "Good" means that the viability was at least 75%.

**[Table 4]**

| Table 4. Viability Measurement Result | |
|---|---|
| Viability | 77% |
| Determination | Good |

### (Examples 13 and 14)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 2% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 2% by weight.

### · Preparation of Additive Reagents

As a stimulating reagent, a tumor necrosis factor (manufactured by Wako Pure Chemical Corporation, hereinafter referred to as "TNF-α") was diluted in pure water. As a luminescent substrate reagent, D-luciferin (manufactured by Wako Pure Chemical Corporation) was diluted with 0.5 mol/L of sodium carbonate solution (manufactured by Wako Pure Chemical Corporation).

### · Preparation of Liquid A with Stimulating Reagent

Separately, Liquid A (with TNF-α) was prepared by adding TNF-α to the above prepared Liquid A to a final concentration of 50 ng/ml.

### · Culture of Cells

NIH3T3/NF B-luc cells (manufactured by Panomics, Inc., hereinafter referred to as "3T3-luc") were cultured for 72 hours in 100 mm dishes using DMEM with 10% calf serum in an incubator. HepG2 was also cultured in the same manner as in Example 1.

### · Preparation of Cell suspension

A cell suspension was prepared in the same manner as in Example 1.

### · Preparation of Extracellular Substrate Solution

Matrigel (manufactured by Corning Inc., registered trademark) was mixed well with serum-free DMEM at 1:1 and stored at 4°C until immediately before use.

### · Preparation of Layered Body

5 × 10⁴ cells of the HepG2 suspended in DMEM with serum stained with the green fluorescent dye described above were seeded in a 96-well multiplate and incubated in an incubator for at least 16 hours. After the incubation, the culture medium was removed with a pipetman, and 16 µl of Liquid A or Liquid A (with TNF-α), which was prepared immediately before use, was added to the culture medium, and Liquid B was added and allowed to gel. After confirming gelation, 50 µl of an extracellular substrate solution was added and incubated at 37°C for 30 minutes. After the incubation, the extracellular substrate solution was removed, and 5 × 10⁴ cells of the 3T3-luc suspended in DMEM with serum were seeded and incubated in an incubator for four hours to prepare a layered body.

### · Evaluation of Stimulation of Layered Body

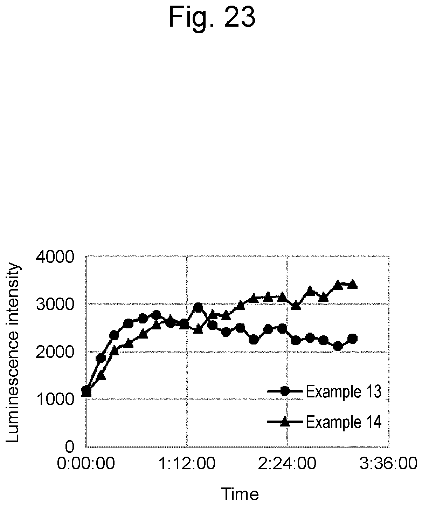

TNF-α was added to the layered body prepared using Liquid A in the gel layer (Example 13) among the above-described layered bodies to a final concentration of 50 ng/ml and incubated in an incubator for two hours. After the incubation, D-luciferin was added to a final concentration of 200 µM, and changes in luminescence intensity were measured every 10 minutes with a plate reader set at 37°C and 5% CO₂ conditions. Similarly, changes in luminescence intensity were also measured for the layered body prepared using Liquid A (TNF-α) as the gel layer (Example 14). The measurement results are indicated in FIG. 23. The luminescence intensity reached the maximum value at one hour in a group in which a medium prepared with Liquid A was added (Example 13), while the luminescence intensity in Example 14 in which Liquid A (with TNF-α) was used increased significantly (p<0.05) even after about three hours, indicating a slow release effect by the inclusion of the stimulating reagent in the gel layer.

### (Reference Example 5)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 2% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 2% by weight.

### · Preparation of Slow Release Drug

Liquid A (with dextran) was prepared by mixing the Liquid A with dextran (molecular weight 570 Da, 10 KDa, and 500 KDa), which was intended for drugs, to reach 1% by weight.

### · Preparation of Slow Release Gel Layer

Equal amounts of Liquid A (with dextran) and Liquid B were added to an insert, and a gel layer was prepared by gelation and incubated in wells with PBS (-) in a microwell plate for 2 hours at 37°C conditions.

### · Measurement of Diffusion Amounts for Different Molecular Weights

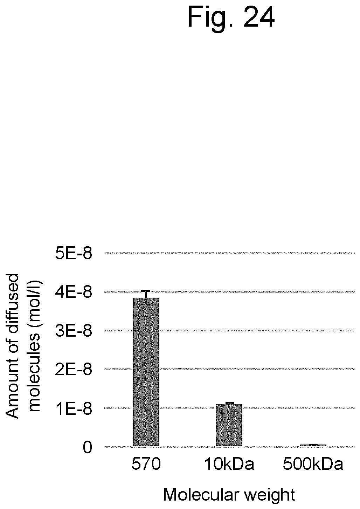

The PBS(-) incubated above was collected, and the fluorescence intensity was measured by a plate reader. The molecular weight of dextran diffused from the gel was calculated from the fluorescence intensity. The results are indicated in Table 5 and FIG. 24. In Table 5, "Good" indicates a diffusion amount of more than 1.5 × 10⁻⁸ mol/l and "Fair" indicates a diffusion amount of 1.0 × 10⁻⁸ mol/l. The diffusion amount was more than 1.5 × 10⁻⁸ mol/l for a molecular weight of 570 Da.

**[Table 5]**

| Table 5. Diffusion amount measurement result | | | |
|---|---|---|---|
| Molecular weight | 570 Da | 10 kDa | 500 kDa |
| Determination | Good | Fair | Fair |

### (Examples 15 and 16)

### · Preparation of Liquid A

In PBS(-)), Tetra-PEG-SH was dissolved, and the resulting solution was filtered through a filter having an average pore size of 0.2 µm, to prepare Liquid A in which the concentration of Tetra-PEG-SH was 2% by weight.

### · Preparation of Liquid B

In PBS(-), Tetra-PEG-maleimide was dissolved, and the resulting solution was filtered through a filter having an average pore size of 0.2 µm, to prepare Liquid B in which the concentration of Tetra-PEG-maleimide was 2% by weight.

### · Culture of Cells

3T3-luc was cultured in a 100 mm dish with DMEM containing 10% calf serum in an incubator for 72 hours.

### · Preparation of Cell Suspension

For the dish in culture, a supernatant was removed using an aspirator. 5 mL of PBS(-) was added to the dish, and the PBS(-) was aspirated off with an aspirator to wash the surface. After repeating the washing process with PBS(-) twice, 2 mL of 0.05% trypsin-0.05% EDTA solution was added to the dish and heated in an incubator for 5 minutes to detach the cells from the dish. After confirming the detachment of the cells by phase contrast microscopy, 4 ml of DMEM with serum was added to the dish. The cell suspension of the dish was transferred to one 15 ml centrifuge tube, and centrifuged, and the supernatant was removed using an aspirator. After the removal, 2 ml of DMEM with serum was added to a centrifuge tube and gently pipetted to disperse the cells to obtain a cell suspension. From the cell suspension, 20 µL of the cell suspension was removed into an Eppendorf tube, and 20 µL of 0.4% trypan blue staining solution was added and pipetted. Twenty µL of the cell suspension was removed from the stained cell suspension and placed on a PMMA plastic slide, and the number of cells in the solution was determined by measuring the number of cells with a Countess.

### · Preparation of Extracellular Substrate Solution

Matrigel was mixed well with serum-free DMEM at 1:1 and stored at 4°C until immediately before use.

### · Preparation of Stimulating Reagent Solution

Hydrogen peroxide water was diluted with PBS(-) to a concentration of 200 mM.

### · Preparation of Layered Body

5 × 10⁴ cells of the 3T3-luc were seeded in a 96-well multiplate and incubated at 37°C for 24 hours. A layered body of Example 15 from which a culture medium was removed and cells were in direct contact with a gel containing a stimulating reagent was prepared by adding 16 µl of the Liquid A (with hydrogen peroxide), adding 16 µl of the Liquid B and allowing the Liquid to gel, adding 50 µl of an extracellular substrate solution, and incubating at 37°C for 30 minutes, followed by removal of the extracellular substrate solution, seeding 5 × 10⁴ cells of the 3T3-luc and incubating at 37°C for 4 hours.

A layered body of Example 16 which had a structure in which a gel layer for stimulation was sandwiched by a gel layer that did not contain a stimulating reagent was prepared as follows. In the process of preparing the layered body of Example 15, after removing the culture medium, 5 µl of the Liquid A was added, an equal amount of the Liquid B was added to confirm gelation, 16 µl of the Liquid A (with hydrogen peroxide) was added, and 16 µl of the Liquid B was added. Subsequently, 5 µl of the Liquid A was added, an equal amount of the Liquid B was added to confirm gelation, 50 µl of an extracellular substrate solution was added and incubated at 37°C for 30 minutes. After the incubation, the extracellular substrate solution was removed, and 5 × 10⁴ cells of the 3T3-luc were seeded and incubated at 37°C for 4 hours to prepare the layered body of Example 16.

### · Evaluation of Stimulation of Layered Body

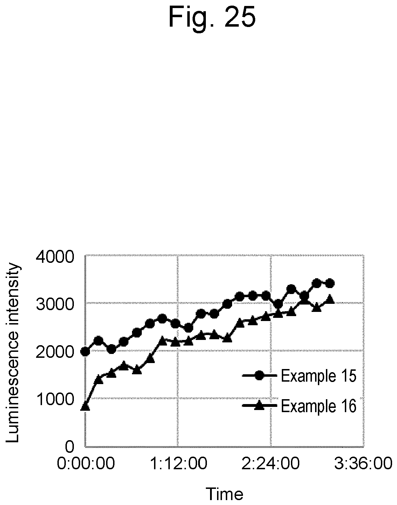

Hydrogen peroxide solution was added to a final concentration of 300 µM in each well and incubated for 6 hours at 37°C and 5% CO₂ conditions. After the incubation, D-luciferin was added to a final concentration of 200 µM, and the luminescence intensity was measured for 3 hours with a plate reader set at 37°C and 5% CO₂ conditions. The measurement results are indicated in FIG. 25. As is clear from FIG. 25, the luminescence intensity of the layered body of Example 15, in which the gel layer prepared with Liquid A (with hydrogen peroxide) was in direct contact with the cell layer, and the layered body of Example 16, in which the gel layer prepared with Liquid A was in contact with the cell layer, differed significantly from the starting point of the measurements, and the difference became smaller with the passage of time. In the case of slow release of a reagent with a small molecular weight, changes over time can be observed by using a structure in which a gel layer sandwiches a gel layer containing a drug.

## Claims

1. An in vitro layered body having a layered structure in which a gel layer containing a hydrogel is disposed between at least two cell layers containing cells of different types from each other, wherein
the hydrogel is
a multi-branched polymer hydrogel formed by a reaction of:
Liquid A containing a multi-branched polymer A, the polymer containing, as a backbone, a polyethylene glycol containing at least three branches, the branches containing one or more electrophilic functional groups in at least one of a side chain(s) and an end(s); and
Liquid B containing a multi-branched polymer B, the polymer containing, as a backbone, a polyethylene glycol containing at least three branches, the branches containing one or more nucleophilic functional groups in at least one of a side chain(s) and an end(s),
a concentration of components derived from the multi-branched polymer A and the multi-branched polymer B in the hydrogel is from 0.6% by weight to 8% by weight, and
a thickness of the layered body ranges from 0.02 mm to 2 mm.

2. A layered body in which a gel layer containing the multi-branched polymer hydrogel is further disposed on top of the layered body according to claim 1.

3. A layered body in which a gel layer containing the multi-branched polymer hydrogel is further disposed at the bottom of the layered body according to claim 1.

4. A layered body in which the side face of the layered body according to claim 1 is further covered with the multi-branched polymer hydrogel.

5. A layered body in which top of the layered body according to claim 4 is further covered with the multi-branched polymer hydrogel.

6. A layered body in which the side face of the layered body according to claim 3 is further covered with the multi-branched polymer hydrogel.

7. A layered body in which top of the layered body according to claim 6 is further covered with the multi-branched polymer hydrogel.

8. The layered body according to any one of claims 1 to 7, wherein the multi-branched polymer A and the multi-branched polymer B are both tetrabranched polymers.

9. A layered body in which the gel layer in the layered body according to any one of claims 1 to 8 contains a drug or a bio-derived liquid factor.

10. The layered body according to claim 9, wherein a vicinity of an interface with the cell layer in the gel layer neither contains a drug nor a bio-derived liquid factor.

11. A method for measuring a cell viability using the layered body according to any one of claims 1 to 10, the method including:
(a) stimulating the layered body;
(b) staining the layered body with a staining reagent for measuring the cell viability;
(c) analyzing the stained layered body by image processing; and
(d) calculating the cell viability based on results obtained from the analysis.

12. A method for evaluating at least one of RNA expression and protein expression using the layered body according to any one of claims 1 to 10, the method including:
(a) stimulating the layered body;
(b) collecting at least two cell layers separately; and
(c) measuring the expression level of at least one of RNA and protein for the collected cell layer.

13. A method for measuring the cell viability using the layered body according to any one of claims 1 to 10, the method including:
(a) stimulating the layered body;
(b) adding a reagent for measuring the cell viability to a culture medium; and
(c) collecting the culture medium to which the reagent is added, and measuring the cell viability.

14. A method for evaluating protein expression using the layered body according to any one of claims 1 to 10, the method including:
(a) stimulating the layered body;
(b) adding a luminescent substrate to the layered body;
(c) measuring the luminescence intensity of the layered body; and
(d) evaluating expression of a protein based on a measurement result.

## Patentansprüche

1. In-vitro-Schichtkörper mit einer Schichtstruktur, in der eine ein Hydrogel enthaltende Gelschicht zwischen mindestens zwei Zellschichten, die Zellen unterschiedlichen Typs enthalten, angeordnet ist, wobei
das Hydrogel
ein mehrfach verzweigtes Polymerhydrogel ist, das durch eine Reaktion von Folgendem gebildet wird:
Flüssigkeit A, die ein mehrfach verzweigtes Polymer A enthält, wobei das Polymer als Rückgrat ein Polyethylenglykol enthält, das mindestens drei Verzweigungen aufweist, wobei die Verzweigungen eine oder mehrere elektrophile funktionelle Gruppen in mindestens einer oder mehreren Seitenketten und einem oder mehreren Enden enthalten; und
Flüssigkeit B, die ein mehrfach verzweigtes Polymer B enthält, wobei das Polymer als Rückgrat ein Polyethylenglykol enthält, das mindestens drei Verzweigungen aufweist, wobei die Verzweigungen eine oder mehrere nukleophile funktionelle Gruppen in mindestens einer oder mehreren Seitenketten und einem oder mehreren Enden enthalten,
die Konzentration der Komponenten, die sich von dem mehrfach verzweigten Polymer A und dem mehrfach verzweigten Polymer B ableiten, in dem Hydrogel 0,6 Gew.-% bis 8 Gew.-% beträgt, und
eine Dicke des Schichtkörpers zwischen 0,02 mm und 2 mm liegt.

2. Schichtkörper, bei dem eine Gelschicht, die das mehrfach verzweigte Polymerhydrogel enthält, zusätzlich an der Oberseite des Schichtkörpers nach Anspruch 1 angeordnet ist.

3. Schichtkörper, bei dem eine Gelschicht, die das mehrfach verzweigte Polymerhydrogel enthält, zusätzlich an der Unterseite des Schichtkörpers nach Anspruch 1 angeordnet ist.

4. Schichtkörper, bei dem die Seitenfläche des Schichtkörpers nach Anspruch 1 ferner mit dem mehrfach verzweigten Polymerhydrogel bedeckt ist.

5. Schichtkörper, bei dem die Oberseite des Schichtkörpers nach Anspruch 4 ferner mit dem mehrfach verzweigten Polymerhydrogel bedeckt ist.

6. Schichtkörper, bei dem die Seitenfläche des Schichtkörpers nach Anspruch 3 ferner mit dem mehrfach verzweigten Polymerhydrogel bedeckt ist.

7. Schichtkörper, bei dem die Oberseite des Schichtkörpers nach Anspruch 6 ferner mit dem mehrfach verzweigten Polymerhydrogel bedeckt ist.

8. Schichtkörper nach einem der Ansprüche 1 bis 7, wobei das mehrfach verzweigte Polymer A und das mehrfach verzweigte Polymer B beide tetrabranchierte Polymere sind.

9. Schichtkörper, bei dem die Gelschicht in dem Schichtkörper nach einem der Ansprüche 1 bis 8 ein Arzneimittel oder einen biologisch gewonnenen flüssigen Faktor enthält.

10. Schichtkörper nach Anspruch 9, wobei die Gelschicht in der Nähe einer Grenzfläche zur Zellschicht weder einen Wirkstoff noch einen biologisch abgeleiteten flüssigen Faktor enthält.

11. Verfahren zur Messung der Lebensfähigkeit einer Zelle unter Verwendung des Schichtkörpers nach einem der Ansprüche 1 bis 10, wobei das Verfahren enthält:
(a) Stimulieren des Schichtkörpers;
(b) Färben des Schichtkörpers mit einem Färbereagenz zur Messung der Lebensfähigkeit der Zellen;
(c) Analysieren des gefärbten Schichtkörpers durch Bildverarbeitung; und
(d) Berechnen der Lebensfähigkeit der Zellen basierend auf den Ergebnissen der Analyse.

12. Verfahren zur Bewertung mindestens einer der Komponenten RNA-Expression und Proteinexpression unter Verwendung des Schichtkörpers nach einem der Ansprüche 1 bis 10, wobei das Verfahren enthält:
(a) Stimulieren des Schichtkörpers;
(b) getrenntes Sammeln von mindestens zwei Zellschichten; und
(c) Messen des Expressionsniveaus von mindestens einer der Komponenten RNA und Protein für die gesammelte Zellschicht.

13. Verfahren zur Messung der Lebensfähigkeit einer Zelle unter Verwendung des Schichtkörpers nach einem der Ansprüche 1 bis 10, wobei das Verfahren enthält:
(a) Stimulieren des Schichtkörpers;
(b) Zugeben eines Reagens zur Messung der Lebensfähigkeit der Zellen zu einem Kulturmedium; und
(c) Sammeln des Kulturmediums, dem das Reagenz zugegeben wurde, und Messen der Lebensfähigkeit der Zellen.

14. Verfahren zur Bewertung der Proteinexpression unter Verwendung des Schichtkörpers nach einem der Ansprüche 1 bis 10, wobei das Verfahren enthält:
(a) Stimulieren des Schichtkörpers;
(b) Zugeben eines lumineszierenden Substrats zu dem Schichtkörper;
(c) Messen der Lumineszenzintensität des Schichtkörpers; und
(d) Auswerten der Expression eines Proteins basierend auf einem Messergebnis.

## Revendications

1. Corps en couches in vitro ayant une structure en couches dans lequel une couche de gel contenant un hydrogel est disposée entre au moins deux couches de cellules contenant des cellules de types différents les unes des autres, dans lequel
l'hydrogel est
un hydrogel polymère multiramifié formé par une réaction de :
Liquide A contenant un polymère A multiramifié, le polymère contenant, comme squelette, un polyéthylène glycol contenant au moins trois branches, les branches contenant un ou plusieurs groupes fonctionnels électrophiles dans au moins l'une d'une ou plusieurs chaînes latérales et d'une ou plusieurs extrémités ; et
Liquide B contenant un polymère B multiramifié, le polymère contenant, comme squelette, un polyéthylène glycol contenant au moins trois branches, les branches contenant un ou plusieurs groupes fonctionnels nucléophiles dans au moins l'une d'une ou plusieurs chaînes latérales et d'une ou plusieurs extrémités,
une concentration de composants dérivés du polymère A multiramifié et du polymère B multiramifié dans l'hydrogel est de 0,6 % en poids à 8 % en poids, et
une épaisseur du corps en couches est comprise entre 0,02 mm et 2 mm.

2. Corps en couches dans lequel une couche de gel contenant l'hydrogel polymère multiramifié est en outre disposée au-dessus du corps en couches selon la revendication 1.

3. Corps en couches dans lequel une couche de gel contenant l'hydrogel polymère multiramifié est en outre disposée au fond du corps en couches selon la revendication 1.

4. Corps en couches dans lequel la face latérale du corps en couches selon la revendication 1 est en outre recouverte de l'hydrogel polymère multiramifié.

5. Corps en couches dans lequel le dessus du corps en couches selon la revendication 4 est en outre recouvert de l'hydrogel polymère multiramifié.

6. Corps en couches dans lequel la face latérale du corps en couches selon la revendication 3 est en outre recouverte de l'hydrogel polymère multiramifié.

7. Corps en couches dans lequel le dessus du corps en couches selon la revendication 6 est en outre recouvert de l'hydrogel polymère multiramifié.

8. Corps en couches selon l'une quelconque des revendications 1 à 7, dans lequel le polymère A multiramifié et le polymère B multiramifié sont tous deux des polymères tétraramifiés.

9. Corps en couches dans lequel la couche de gel dans le corps en couches selon l'une quelconque des revendications 1 à 8 contient un médicament ou un facteur liquide d'origine biologique.

10. Corps en couches selon la revendication 9, dans lequel le voisinage d'une interface avec la couche cellulaire dans la couche de gel ne contient ni médicament ni facteur liquide d'origine biologique.

11. Procédé pour mesurer une viabilité cellulaire utilisant le corps en couches selon l'une quelconque des revendications 1 à 10, le procédé comprenant :
(a) la stimulation du corps en couches ;
(b) la coloration du corps en couches avec un réactif de coloration pour mesurer la viabilité cellulaire ;
(c) l'analyse du corps en couches coloré par traitement d'image ; et
(d) le calcul de la viabilité cellulaire sur la base des résultats obtenus à partir de l'analyse.

12. Procédé pour évaluer au moins l'une parmi l'expression d'ARN et l'expression de protéine en utilisant le corps en couches selon l'une quelconque des revendications 1 à 10, le procédé comprenant :
(a) la stimulation du corps en couches ;
(b) la collecte séparée d'au moins deux couches de cellules ; et
(c) la mesure du niveau d'expression d'au moins l'un parmi l'ARN et la protéine pour la couche cellulaire collectée.

13. Procédé pour mesurer la viabilité cellulaire en utilisant le corps en couches selon l'une quelconque des revendications 1 à 10, le procédé comprenant :
(a) la stimulation du corps en couches ;
(b) l'ajout d'un réactif pour mesurer la viabilité cellulaire à un milieu de culture ; et
(c) la collecte du milieu de culture auquel le réactif est ajouté et la mesure de la viabilité cellulaire.

14. Procédé pour évaluer l'expression d'une protéine utilisant le corps en couches selon l'une quelconque des revendications 1 à 10, le procédé comprenant :
(a) la stimulation du corps en couches ;
(b) l'ajout d'un substrat luminescent au corps en couches ;
(c) la mesure de l'intensité de luminescence du corps en couches ; et
(d) l'évaluation de l'expression d'une protéine sur la base d'un résultat de mesure.
